(19) Europäisches Patentamt
European Patent Office
Office européen des brevets

(11) EP 0 745 598 B1

(12) # FASCICULE DE BREVET EUROPEEN

(45) Date de publication et mention de la délivrance du brevet:
**22.08.2001 Bulletin 2001/34**

(21) Numéro de dépôt: **96401156.3**

(22) Date de dépôt: **30.05.1996**

(51) Int Cl.[7]: **C07D 319/18**, C07D 307/79, C07D 321/10, C07D 409/12, C07D 405/04, C07D 409/14, A61K 31/495, A61K 31/435

(54) **Nouveaux composés de la pipérazine, de la pipéridine et de la 1,2,5,6-tétrahydropyridine, leur procédé de préparation et les compositions pharmaceutiques les contenant**

Piperazin-, Piperidin- und 1,2,5,6-Tetrahydropyridinderivate, Verfahren zu ihrer Herstellung und diese enthaltende pharmazeutische Zusammensetzungen

Piperazine, piperidine and 1,2,5,6-tetrahydropyridine derivatives, proces for their preparation and pharmaceutical compositions containing them

(84) Etats contractants désignés:
**AT BE CH DE DK ES FI FR GB GR IE IT LI LU NL PT SE**

(30) Priorité: **31.05.1995 FR 9506436**

(43) Date de publication de la demande:
**04.12.1996 Bulletin 1996/49**

(73) Titulaire: **ADIR ET COMPAGNIE**
**92415 Courbevoie Cédex (FR)**

(72) Inventeurs:
- **Peglion, Jean-Louis**
**78110 Le Vesinet (FR)**
- **Dessinges, Aimée**
**94320 Thiais (FR)**
- **Goument, Bertrand**
**78220 Viroflay (FR)**
- **Millan, Mark**
**75017 Paris (FR)**
- **Newman-Tancredi, Adrian**
**78230 Le Pecq (FR)**
- **Gobert, Alain**
**93200 Saint Denis (FR)**

(56) Documents cités:
**EP-A- 0 490 772**
**EP-A- 0 574 313**
**EP-A- 0 529 462**
**EP-A- 0 634 398**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 745 598 B1

## Description

La présente invention a pour objet des nouveaux composés de la pipérazine, de la pipéridine et de la 1, 2, 5, 6-tétrahydropyridine, leur procédé de préparation et les compositions pharmaceutiques les contenant.

Elle concerne plus particulièrement les composés de formule I :

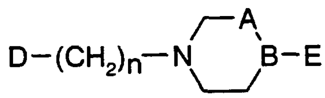

(I)

dans laquelle

A-B représente : $CH_2$-CH, CH=C ou $CH_2$-N,
n représente zéro ou un nombre entier de 1 à 6 inclus,
D représente l'un des systèmes bicycliques suivants :

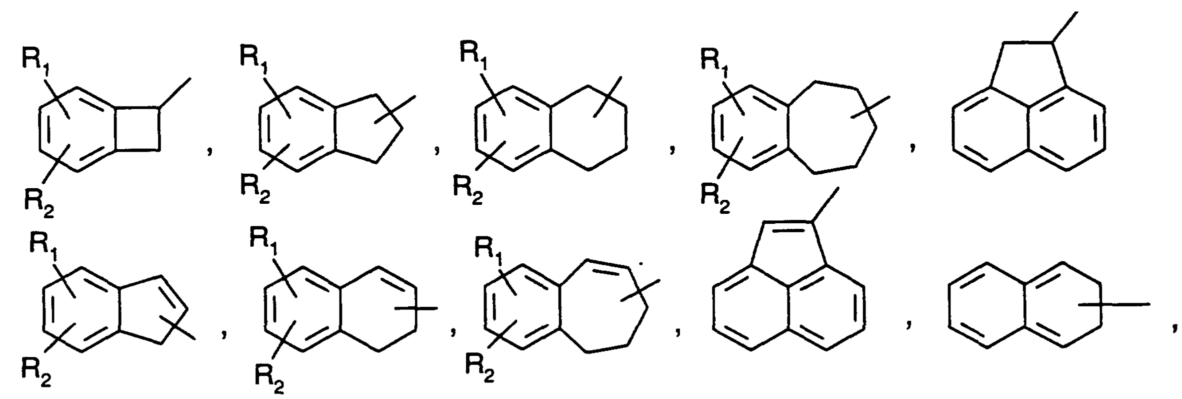

, , , , , , , , ,

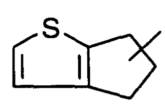 , 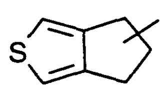 , 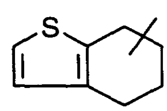 ,

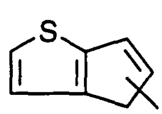 , 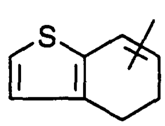 et 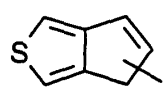

dans lesquels :

$R_1$ et $R_2$, identiques ou différents, représentent chacun un atome d'hydrogène ou d'halogène, un radical alkyle ou alkoxy en chaîne droite ou ramifiée ayant de 1 à 5 atomes de carbone inclus ou un radical hydroxy ; et
E représente un des hétérocycles suivants :

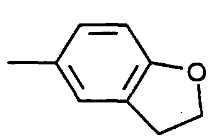 , 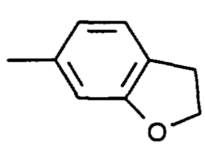 , 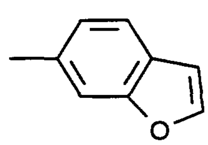 ,

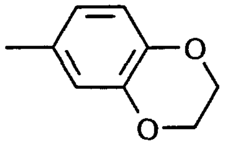 , 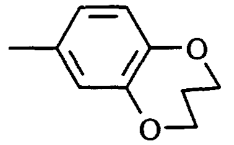 , 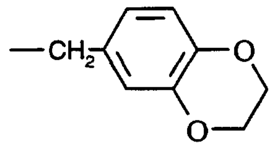 , 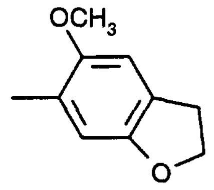 ,

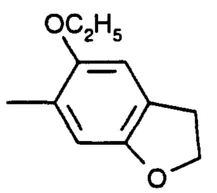 , 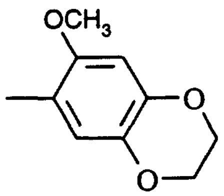 , 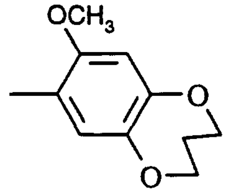 et 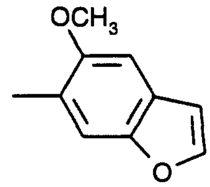 ,

à la condition toutefois que E ne représente pas :

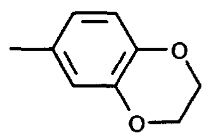

lorsque D représente :

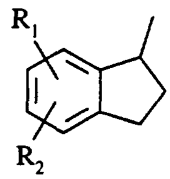 ,

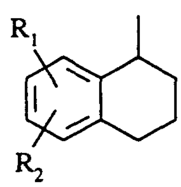 ou 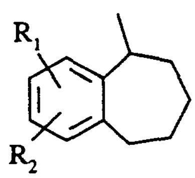 .

La présence de carbones asymétriques implique que les molécules de l'invention existent sous la forme de mélange racémique ou racémate et d'isomères optiques ou énantiomères également inclus dans la présente invention. De plus les composés de l'invention peuvent former, avec des acides pharmaceutiquement acceptables, des sels d'acides organiques ou minéraux, qui font aussi partie de la présente invention.

Le système dopaminergique est impliqué dans un nombre important de maladies du système nerveux central, liées aussi bien à une hyperactivité (telle que, par exemple, la schizophrénie) qu'à une hypoactivité (comme, par exemple, la maladie de Parkinson) de ce système. La dépression, les troubles de l'impulsion et de la mémoire font aussi partie des maladies pour lesquelles il a été possible de démontrer le rôle joué par la dopamine dans leur étiologie. Jusqu'à présent le traitement de ces maladies était assuré par des bloqueurs dopaminergiques $D_2$ (pour les troubles liés à l'hyperactivité) et par des activateurs dopaminergiques $D_2$ (pour les troubles liés à l'hypoactivité). Toutefois les traitements par les neuroleptiques conventionnels, qui sont des bloqueurs des récepteurs dopaminergiques $D_2$, sont associés à de nombreux effets secondaires : diskynésie tardive, syndrome neuroleptique malin, hyperprolactinémie et aménorrhée. De plus les stimulants des récepteurs dopaminergiques $D_2$ provoquent des nausées et des effets secondaires cardiovasculaires et moteurs gênants. Récemment trois autres récepteurs dopaminergiques ont été découverts en plus des récepteurs $D_1$ et $D_2$ déjà connus : le $D_3$ (P. Sokoloff et al, Nature, 1990, 347, 147), le $D_4$ (Van Tol

et al, Nature, 1991, 350, 610) et le $D_5$ (Sunhara et al, Nature, 1991, 350, 614). La présente invention concerne plus particulièrement des ligands, agonistes ou antagonistes des récepteurs $D_4$, possédant une bonne sélectivité vis à vis des autres récepteurs dopaminergiques et notamment $D_2$, ce qui confère à ces produits des propriétés thérapeutiques intéressantes tout en étant dépourvus des effets secondaires connus des ligands $D_2$ et en raison de l'absence relative de récepteur $D_4$ dans l'hypophyse et dans les structures de ganglia basalis.

Il faut également mentionner qu'un renforcement de la transmission dopaminergique corticale joue un rôle clé dans le traitement des symptômes déficitaires de la schizophrénie.

L'état antérieur de la technique le plus proche de la présente invention, concerne des composés de la 1-(2,3-dihydro-1,4-benzodioxin-6-yl) pipérazine, décrits dans les brevets USP 5242925 (agonistes / antagonistes sérotoninergiques), EP 300908 (anti-arythmiques), EP 072960 et EP 072961 (anti-allergiques). Ces brevets n'incluent, ni ne suggèrent nullement les dérivés objet de la présente invention et ne sauraient influencer la brevetabilité de la présente demande.

Les brevets EP 0490772 et EP 0574313 décrivent de nouvelles pipérazines 1,4-disubstituées qui sont revendiquées pour leur activité antagoniste $5HT_{1A}$, les rendant ainsi utiles pour le traitement des maladies du système nerveux central et les maladies neuro-endocriniennes.

Les composés de la présente invention se différencient donc des composés de l'état antérieur de la technique non seulement par leur structure chimique mais aussi au niveau de leurs activités pharmacologiques et thérapeutiques. Ces activités ont été mises en évidence :

*in vitro* : par des études de binding sur récepteurs clonés $D_2$ et $D_4$ humains, et

*in vivo* :

- **a) sur des modèles pharmacologiques :**
  - par des études de synthèse (turnover) de la dopamine dans les structures suivantes : cortex frontal (voie mésocorticale), noyau accumbens et tubercule olfactif (voie mésolimbique), striatum (voie nigrostriatale). Les antagonistes dopaminergiques fonctionels provoquent une augmentation de la synthèse de dopamine dans ces structures.
  - par des études de dialyse, dans les structures ci-dessus mentionnées, au cours desquelles les produits de l'invention sont caractérisés en fonction de leur effets sur l'activité dopaminergique, noradrénergique ou sérotoninergique. Une augmentation sélective de la libération de dopamine dans le cortex frontal par rapport au noyau accumbens et au striatum peut laisser prévoir des effets thérapeutiques de type antidepresseur, antipsychotique et pro-mnésiant.

    Les activités ci-dessus ont été confirmées.
- **b) sur des modèles thérapeutiques :**
  et notamment sur les tests :
  - d'inhibition de la verticalisation induite par l'apomorphine chez les souris (propriétés antipsychotiques)
  - d'inhibition de l'agressivité chez les souris isolées (propriétés anti-impulsives et anxiolytiques).

D'autre part l'absence d'effets secondaires a été mise en évidence notamment par une absence d'activité dans le test :

- d'induction de catalepsie chez le rat.

Ainsi, agissant comme ligands sélectifs des récepteurs $D_4$ les produits de l'invention peuvent être utilisés dans la prévention ou les maladies liées à un dysfonctionnement du système dopaminergique. Plus particulièrement leur utilité comme anti-psychotique et anti-dépresseur, dans le traitement des troubles des impulsions, de la mémoire et comme anxiolytique est revendiquée en relation avec leur activité dans les tests cités ci-dessus.
L'invention s'étend également au procédé de préparation des composés de formule I caractérisé en ce que l'on condense :

- un composé de formule II :

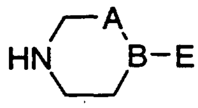

(II)

dans laquelle A-B et E ont la signification précédemment définie,

- avec un composé de formule III :

$$D\text{-}(CH_2)n\text{-}X \qquad (III)$$

dans laquelle n et D ont les significations précédemment définies et X représente un atome d'halogène, ou un radical mésyloxy ou tosyloxy.
La condensation s'effectue de façon particulièrement adéquate en opérant dans un solvant approprié tel que, par exemple, la méthyl éthylcétone, la méthyl isobutylcétone, le toluène, le diméthylformamide ou le diméthylacétamide, en présence d'un accepteur de l'acide formé au cours de la réaction, à une température de 20 à 150° C. Comme accepteur, on peut employer par exemple un carbonate de métaux alcalins comme le carbonate de sodium ou une amine tertiaire comme la triéthylamine.

De plus, les composés de formule I dans laquelle n prend les significations autres que zéro, c'est-à-dire les composés répondant plus précisément à la formule I' :

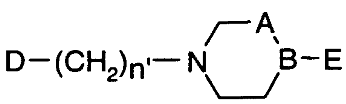

(I')

dans laquelle A-B, D et E ont les significations précédemment définies et n' représente un nombre entier de 1 à 6, ont également été préparés selon une variante du procédé précédent, caractérisé en ce que l'on condense :

- un composé de formule II précédemment définie
  avec
- un composé de formule IV :

$$D\text{-}(CH_2)_{n'\text{-}1}\text{-}CO_2H \qquad (IV)$$

dans laquelle :

D et n' ont les significations précédemment définies,
et l'on réduit l'amide ainsi obtenue de formule V :

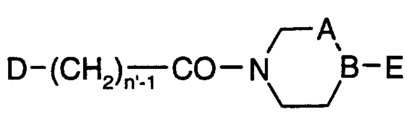

(V)

dans laquelle
A-B, D, E et n' ont les significations précédemment définies.
La condensation des composés II et IV s'effectue de façon particulièrement adéquate en opérant dans un solvant approprié comme par exemple le chlorure de méthylène, en présence de carbonyldiimidazole.
La réduction de l'amide V s'effectue avantageusement au moyen d'un hydrure double de lithium et d'aluminium dans l'éther ou le tétrahydrofurane ou bien au moyen de borane diméthylsulfure dans le tétrahydrofurane, ou bien encore d'alcoxyaluminohydrure de sodium dans le toluène tel que le Red Al®.
Ce dernier procédé de préparation des dérivés I' est également inclus dans la présente invention.

De plus les amides de formule V sont des produits intermédiaires nouveaux qui font, à ce titre, partie de la présente invention.

Les matières premières de formules II, III et IV sont soit des produits connus, soit des produits préparés à partir de composés connus selon des procédés connus, comme précisés dans les exemples ci-après.

Les composés de formule I donnent des sels avec les acides physiologiquement tolérables. Ces sels sont également inclus dans la présente invention.

La présente invention a également pour objet les compositions pharmaceutiques contenant comme principe actif un composé de formule générale I ou un de ses sels physiologiquement tolérables, mélangé ou associé à un excipient pharmaceutique approprié, comme par exemple, le glucose, le lactose, le talc, l'éthylcellulose, le stéarate de magnésium ou le beurre de cacao. Les compositions pharmaceutiques ainsi obtenues se présentent généralement sous forme dosée et peuvent contenir de 0,1 à 100 mg de principe actif. Elles peuvent revêtir, par exemple, la forme de comprimés, dragées, gélules, suppositoires, solutions injectables ou buvables et être selon les cas, administrées par voie orale, rectale ou parentérale à la dose de 0,1 à 100 mg de principe actif 1 à 3 fois par jour.

Les exemples suivants illustrent la présente invention, les points de fusion étant déterminés à la platine chauffante de Kofler (K) éventuellement sous microscope (M.K.).

**Exemple 1**

1-(benzocyclobutan-1-yl methyl)4-(2,3-dihydrobenzo-1,4-dioxin-6-yl) pipérazine

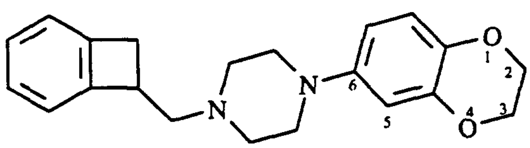

On mélange 2,26 g (7,8 $10^{-3}$ M) de tosylate de 1-hydroxyméthyl benzocyclobutane, 1,7 g (7,8 $10^{-3}$ M) de 4-(2,3-dihydrobenzo-1,4-dioxin-6-yl) pipérazine et 2,16 g (15,6 $10^{-3}$ M) de $K_2CO_3$ dans 50 ml de méthyl isobutylcétone. On chauffe ce mélange à 100° C pendant 8h puis le refroidit. On concentre et reprend le résidu par de l'eau et de l'acétate d'éthyle. On décante, puis extrait la phase organique par HCl 1N. On basifie la phase aqueuse par NaOH 1N puis extrait au chlorure de méthylène et sèche sur $MgSO_4$. L'huile obtenue est purifiée par flash-chromatographie (éluant $CH_2Cl_2$ / $CH_3OH$ : 95/5). On obtient 1 g (Rdt = 38 %) d'une huile qui correspond au produit titre dont on prépare le dichlorhydrate dans l'acétonitrile. PF : 242 - 245° C.

**Exemple 2**

4-(2,3-dihydrobenzo-1,4-dioxin-6yl)-1-(indan-2-yl)pipérazine

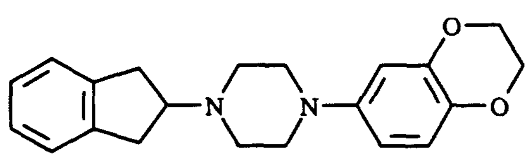

Préparé de la même façon que dans l'exemple 1 mais en utilisant le tosylate de l'indan-2-ol à la place du tosylate du 1-hydroxyméthyl benzocyclobutane, le produit titre obtenu fond à 183 -185° C.

**Exemple 3**

1-[2-(benzocyclobutan-1-yl) ethyl]-4-(2,3-dihydro-5-methoxy benzofuran-6-yl) pipérazine

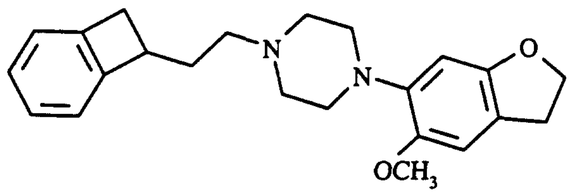

2,21 g (8,2 mM) de 4-(2,3-dihydro-5-methoxy benzofuran-6-yl) pipérazine (préparation 1), 1,72 g (8,2 mM) de 2-(benzocyclobutan-1-yl)-1-bromoéthane et 3,48 g (32,8 mM) de carbonate de sodium dans 33 ml de méthyl isobutylcétone sont portés à reflux pendant 14 h. On évapore à sec, reprend par 200 ml d'acétate d'éthyle et 100 ml de soude N, décante, et lave la phase organique par 100 ml d'une solution saturée en chlorure de sodium. Après séchage sur Mg $SO_4$ et évaporation le résidu obtenu est chromatographié sur silice (éluant : $CH_2Cl_2/CH_3OH$ : 98/2) pour donner 2,1 g de produit attendu. Par addition d'une solution à 2 % d'acide fumarique dans l'éthanol on obtient 1,7 g de fumarate de produit titre. P.F.: 193 - 194° C.

**Exemple 4**

1-(2,3-dihydro-5-methoxy benzofuran-6-yl)-4-[2-(napht-1-yl)éthyl]pipérazine

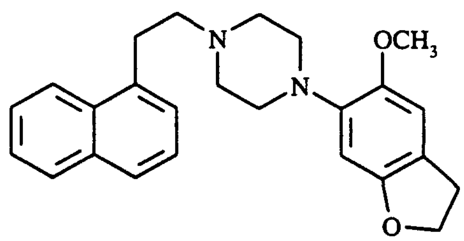

Préparée de la même façon que le produit de l'exemple 3 mais en utilisant le 2-(napht-1-yl) 1-bromoéthane à la place du 2-(benzocyclobutan-1-yl)-1-bromoéthane. Le fumarate du produit titre obtenu fond à 177-179°C, après recristallisation de l'éthanol.

**Exemple 5**

4-[2-(benzocycloheptan-1-yl)éthyl]-1-(2,3-dihydro-5-methoxy benzofuran-6-yl)pipérazine

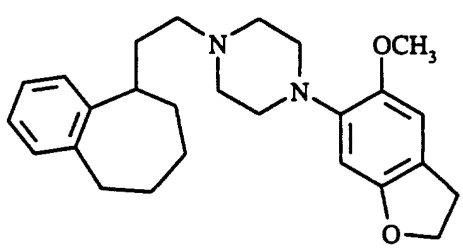

Préparée de la même façon que le produit de l'exemple 3 mais en utilisant le mésylate du 2-(benzocycloheptan-1-yl)éthanol à la place du 2-(benzocyclobutan-1-yl)-1-bromoéthane. Le fumarate du produit titre obtenu fond à

225-227°C après recristallisation de l'éthanol.

**Exemple 6**

4-(2,3-dihydrobenzo-1,4-dioxin-6-yl)-1-(indan-2-yl methyl) pipérazine

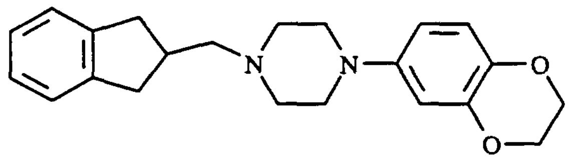

*Stade 1. "Amide"*

A 4,9 g (29,5 mM) d'acide indan-2-yl carboxylique dissous dans 50 ml de chlorure de méthylène, on ajoute en une fois 4,9 g (29,5 mM) de carbonyldimidazole. On laisse une heure en contact après la fin du dégagement gazeux, puis on ajoute en un goutte à goutte rapide 6,4 g (29,5 mM) de 4-(2,3-dihydrobenzo-1,4-dioxin-6-yl) pipérazine en solution dans 50 ml de chlorure de méthylène. On laisse en contact la nuit, transvase en ampoule à décanter, extrait à HCl 1N. Les phases acides sont basifiées à froid et extraites à l'acétate d'éthyle. On obtient 8,3 g d'amide attendu (Rdt = 69 %) que l'on utilise sans autre purification.

*Stade 2. Produit titre.*

Une solution de 8 g (21,9 mM) de l'amide précédemment obtenu dans 100 ml de THF est coulée sur 0,8 g de $LiAlH_4$ en suspension dans 30 ml de THF. On laisse la nuit en contact. On décompose successivement par $H_2O$ (0,54 ml), NaOH à 20 % (0,44 ml) et $H_2O$ (2 ml). On filtre le précipité, le rince au THF et évapore pour obtenir une huile qui correspond au produit désiré. Par addition lente d'une solution d'éther chlorhydrique à la base en solution dans l'acétonitrile on obtient 1,5 g du dichlorhydrate du produit titre. P.F. : 220 - 222° C.

**Exemple 7**

1-(indan-2-yl méthyl)-4-(2,3-dihydro-5-méthoxy benzofuran-6-yl)pipérazine

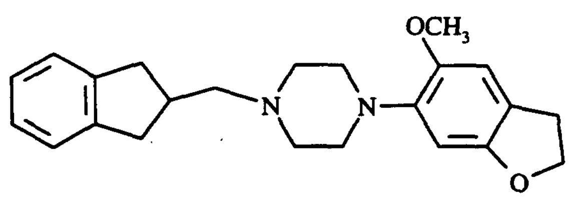

Le produit titre a été obtenu de la même façon que le produit de l'exemple 6 mais en utilisant au stade 1 la 4-(2,3-dihydro-5-méthoxy benzofuran-6-yl)pipérazine (préparation 1) à la place de la 4-(2,3-dihydrobenzo-1,4-dioxin-6-yl)pipérazine. Le chlorhydrate du produit titre, obtenu par addition lente d'une solution d'éther chlorhydrique à la base en solution dans l'éther fond à 201-204 °C.

**Exemple 8**

4-[2-(benzocyclohept-1-en-1-yl)éthyl]-1-(2,3-dihydro-5-méthoxy benzofuran-6-yl)pipérazine

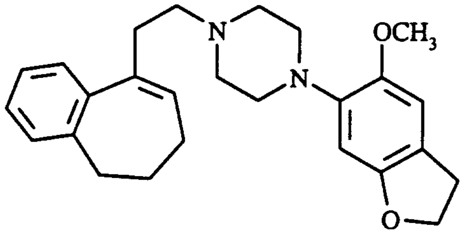

Obtenue de la même façon que le produit de l'exemple 6 mais en utilisant au stade 1 la 4-(2,3-dihydro-5-méthoxy benzofuran-6-yl)pipérazine à la place de la 4-(2,3-dihydrobenzo-1,4-dioxin-6-yl)pipérazine d'une part et l'acide 2-(benzocyclohept-1-en-1-yl) acétique à la place de l'acide indan-2-yl carboxylique d'autre part.
Le fumarate du produit titre fond à 207-209°C après recristallisation de l'éthanol.

**Exemple 9**

1-[2-(benzocyclobutan-1-yl)éthyl]-4-(2,3-dihydrobenzo-1,4-dioxin-6-yl) pipérazine

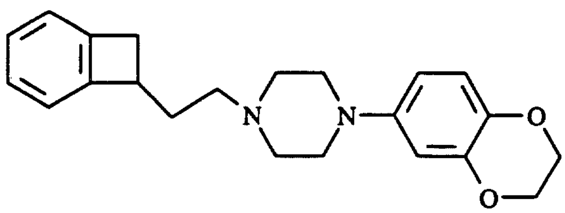

*Stade 1. "Amide"*
Identique au stade 1 de l'exemple 6 mais en utilisant l'acide benzocyclobutan-1-yl acétique à la place de l'acide indan-2-yl carboxylique.

*Stade 2. Produit titre.*
A 7,9 mM de l'amide obtenue ci-dessus dans 150 ml de THF anhydre sont ajoutés goutte à goutte 7,92 ml (79,2 mM) de borane-diméthylsulfure . On porte à reflux pendant 6 h. Après retour à tempéraure ambiante on décompose en coulant goutte à goutte 16 ml de méthanol, puis en portant 3h à reflux. Après évaporation des solvants on obtient une huile qui correspond au produit titre (Rdt = 93 %). Le chlorhydrate fond à 200 - 204° C.

**Exemple 10**

1-[3-(benzocyclobutan-1-yl)propyl]-4-(2,3dihydrobenzo-1,4-dioxin-6-yl) pipérazine

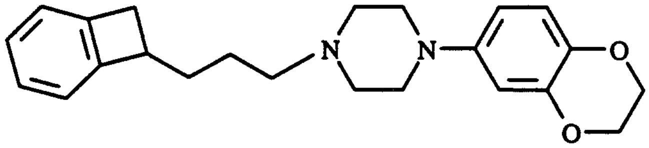

Préparée de la même façon que le produit titre de l'exemple 9 mais en utilisant pour préparer l'amide le mode opératoire du stade 1 de l'exemple 6 dans lequel l'acide indan-2-yl carboxylique a été remplacé par l'acide 3-(benzocyclobutan-1-yl) propionique (Rdt = 79 %). Le dichlorhydrate fond à 196 - 199° C.

**Exemple 11**

4-(2,3-dihydrobenzo-1,4-dioxin-6-yl)-1-(indan-2-yl méthyl) pipéridine

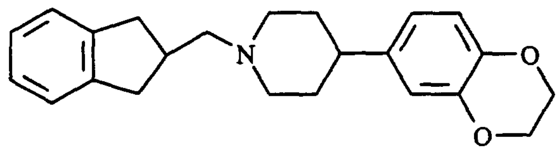

*Stade 1. "Amide"*
Identique au stade 1 de l'exemple 6 mais en utilisant la 4-(2,3-dihydrobenzo-1,4-dioxin-6-yl) pipéridine (préparation 2) à la place de la 4-(2,3-dihydrobenzo-1,4-dioxin-6-yl) pipérazine. Après une flash-chromatographie sur silice avec un éluant composé du mélange $CH_2Cl_2/CH_3COOC_2H_5$ : 95/5 on obtient l'amide attendu avec un rendement de 61 %.

*Stade 2. Produit titre.*
Identique au stade 2 de l'exemple 9. Le dichlorhydrate du produit titre fond à 218 - 220° C. (Rdt = 42 %).

**Exemple 12**

1-(2,3-dihydro-5-méthoxy benzofuran-6-yl)-4-[2-(1,2,3,4-tétrahydro naplyhalen-1-yl)éthyl]pipérazine

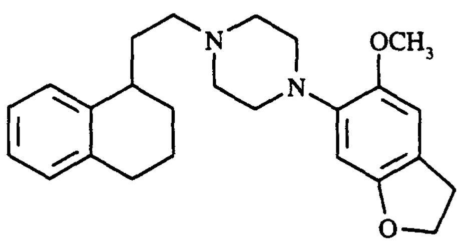

Préparée comme décrit dans l'exemple 9 à partir de l'acide 1,2,3,4-tétrahydro naphthalen-1-yl acétique et de la 4-(2,3-dihydro-5-méthoxy benzofuran-6-yl) pipérazine. Le fumarate du produit titre fond à 219-220°C après recristallisation de l'éthanol.

**Exemple 13**

1-(2,3-dihydro-5-éthoxy benzofuran-6-yl)-4-[2-(indan-2-yl)méthyl]pipérazine

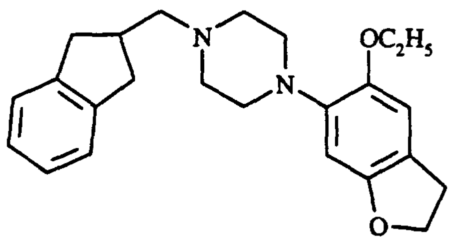

Préparée comme décrit dans l'exemple 9, à partir de la 4-(2,3-dihydro-5-éthoxy benzofuran-6-yl) pipérazine (préparation 4). Le fumarate du produit titre fond à 183-185°C (éthanol).

**Exemple 14**

1-[3-(benzocyclobutan-1-yl) propyl]-4-(benzo-1,5-dioxépin-7-yl)-pipérazine

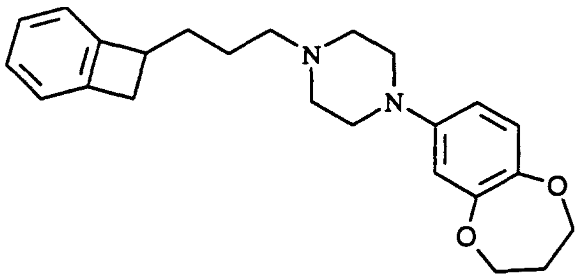

Préparée comme décrit dans l'exemple 9 à partir de l'acide 3-(benzocyclobutan-1-yl) propionique, et de la 4-(benzo-1,5-dioxépin-7-yl) pipérazine. Le fumarate du produit titre fond à 168-170°C (éthanol).

**Exemple 15**

4-(benzo-1,5-dioxepin-7-yl)-1-[(indan-2-yl)méthyl]-pipérazine

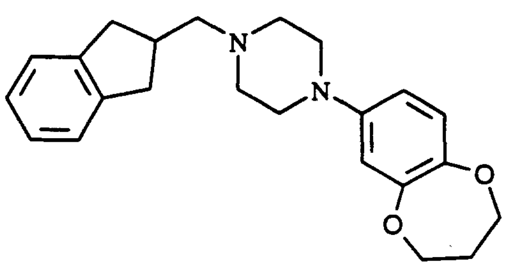

Préparée comme décrit dans l'exemple 9 à partir de la 4-(benzo-1,5-dioxepin-7-yl) pipérazine. L'hémifumarate du produit titre fond à 179-181°C (éthanol).

**Exemple 16**

4-[(2,3-dihydro benzo-1,4-dioxin-6-yl)méthyl]-1-[(indan-2-yl)méthyl]-pipérazine

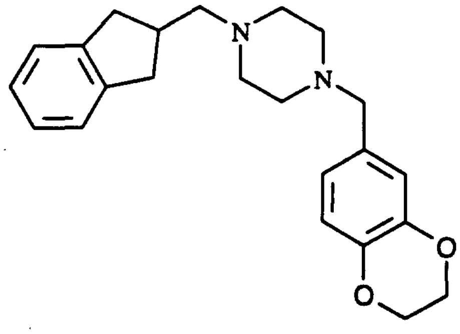

Préparée comme décrit dans l'exemple 9 à partir de la 4-[(2,3-dihydro benzo-1,4-dioxin-6-yl)méthyl]pipérazine. Le difumarate du produit titre fond à 217-220°C (éthanol).

**Exemple 17**

4-[(2,3-dihydro-5-méthoxybenzofuran-6-yl)]-1-[(4,5,6,7-tétrahydro-5-yl)méthyl]pipérazine

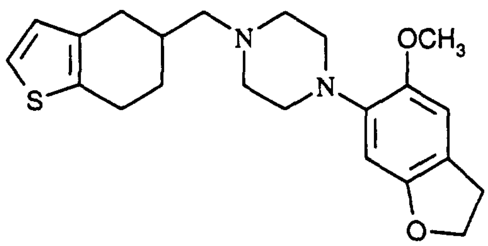

Préparée comme décrit dans l'exemple 9, à partir de l'acide 4,5,6,7-tétrahydro benzo[b]thién-5-yl carboxylique et de la 4-(2,3-dihydro-5-méthoxybenzofuran-6-yl) pipérazine. Le fumarate du produit titre fond à 198-200°C (éthanol).

**Exemple 18**

1-(2,3-dihydro-benzo-1,4-dioxin-6-yl)-4-[2-(napht-1-yl)éthyl]pipérazine

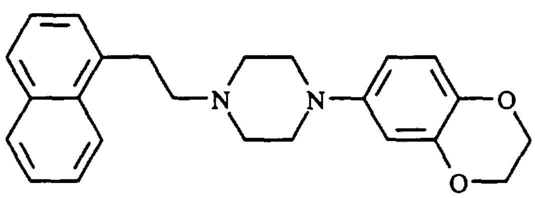

Préparée comme décrit dans l'exemple 9, à partir de l'acide napht-1-yl acétique. Le dichlorhydrate du produit titre fond à 223-232°C (méthanol).

**Exemple 19**

1-[(cyclopenta[b]thién-5-yl)méthyl] 4-(2,3-dihydro-5-méthoxy benzofuran-6-yl) pipérazine

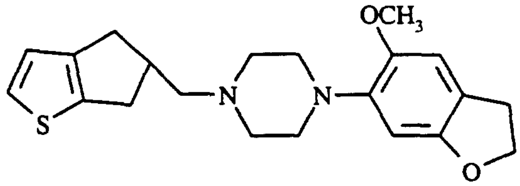

Préparée comme décrit dans l'exemple 9, à partir de l'acide (cyclopenta[b]thién-5-yl) carboxylique (préparation 8) et de la 4-(2,3-dihydro-5-méthoxy benzofuran-6-yl) pipérazine. Le fumarate du produit titre fond à 186-190°C (éthanol).

**Exemple 20**

1-[(cyclopenta[c]thién-5-yl)méthyl]4-(2,3-dihydro-5-méthoxy benzofuran-6-yl)pipérazine

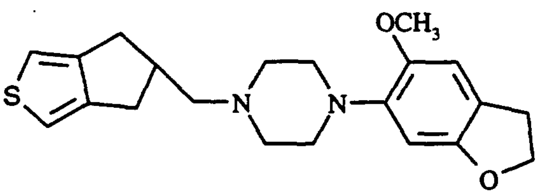

Préparée comme décrit dans l'exemple 9, à partir de l'acide (cyclopenta[c]thién-5-yl) carboxylique (préparation 7) et de la 4-(2,3-dihydro-5-méthoxy benzofuran-6-yl) pipérazine. Le produit titre fond à 156-158°C.

**Exemple 21**

4-(2,3-dihydro-benzo-1,4-dioxin-6-yl)-1-[2-(napht-1-yl)éthyl]-pipéridine

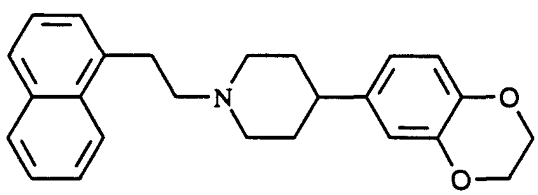

Préparée comme décrit dans l'exemple 11 mais en utilisant au stade 1 l'acide napht-1-yl acétique à la place de l'acide indan-2-yl carboxylique. Le chlorhydrate du produit titre fond à 220-223°C (cyanure de méthyle).

**Exemple 22**

1-[(acénaphtén-1-yl)méthyl]-4-(2,3-dihydro-5-méthoxy benzofuran-6-yl)pipérazine

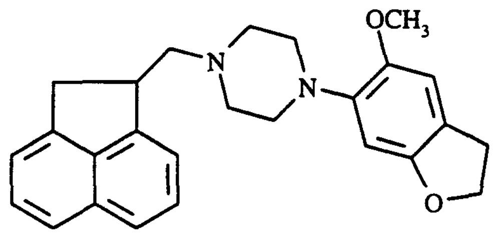

Préparée comme décrit dans l'exemple 9, à partir de l'acide acénaphtén-1-yl carboxylique et de la 4-(2,3-dihydro-5-méthoxy benzofuran-6-yl) pipérazine. Le fumarate du produit titre fond à 226-228°C (éthanol).

**Exemple 23**

4-(2,3-dihydro-7-méthoxy benzo-1,4-dioxin-6-yl)-1-[(indan-2-yl)méthyl] pipérazine

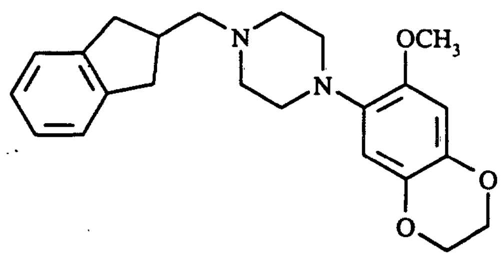

Préparée comme décrit dans l'exemple 9, à partir de la 4-(2,3-dihydro-7-méthoxy benzo-1,4-dioxin-6-yl) pipérazine (préparation 5). Le fumarate du produit titre fond à 176-178°C (éthanol).

**Exemple 24**

4-(2,3-dihydro benzo-1,4-dioxin-6-yl)-1-[(1,2,3,4-tétrahydro napht-2-yl)méthyl]pipérazine

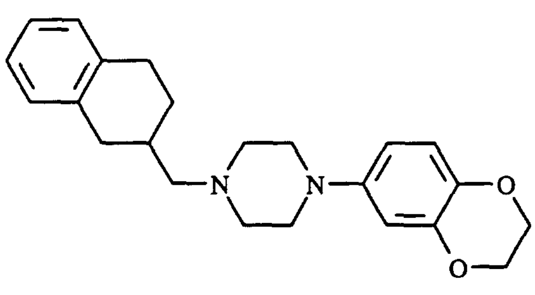

Préparée comme décrit dans l'exemple 9, à partir de l'acide 1,2,3,4-tétrahydronapht-2-yl carboxylique. Le chlorhydrate du produit titre fond à 226-229°C (méthanol).

**Exemple 25**

4-(2,3-dihydro-5-méthoxy benzofuran-6-yl)-4-[(1,2,3,4-tétrahydro napht-2-yl)méthyl]pipérazine

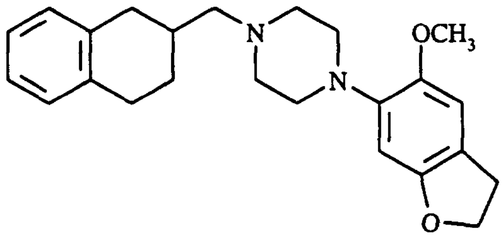

Préparée comme décrit dans l'exemple 9, à partir de l'acide 1,2,3,4-tétrahydronapht-2-yl carboxylique et de la 4-(2,3-dihydro-5-méthoxy benzofuran-6-yl) pipérazine. Le fumarate du produit titre fond à 219-221°C (éthanol).

**Exemple 26**

1-(acénaphtén-1-yl méthyl)-4-(2,3-dihydro benzo-1,4-dioxin-6-yl) pipérazine

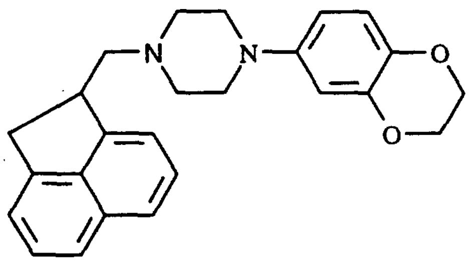

Préparée comme décrit dans l'exemple 9, à partir de l'acide acénaphtén-1-yl carboxylique. Le chlorhydrate du produit titre fond à 192-196°C (éther).

**Exemple 27 :**

1-[(indan-2-yl)méthyl]-4-(8-méthoxy benzo-1,5-dioxépin-7-vl)pipérazine

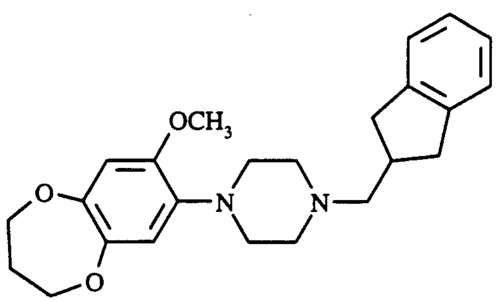

Préparée comme décrit dans l'exemple 9, à partir de la 4-(8-méthoxy benzo-1,5-dioxépin--7-yl) pipérazine (Préparation 6). Le produit titre fond à 120-122°C (éthanol).

**Exemple 28 :**

4-(2,3-dihydrobenzofuran-5-yl)-1-(indan-2-yl méthyl) pipérazine

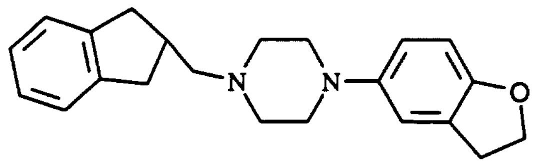

Préparée comme décrit dans l'exemple 9, à partir de la 4-(2,3-dihydro-benzofuran-5-yl) pipérazine. Le fumarate du produit titre fond à 180-182°C (éthanol).

**Exemple 29 :**

4-(2,3-dihydro-5-méthoxy benzofuran-6-yl)-1-(indan-1-yl méthyl) pipérazine

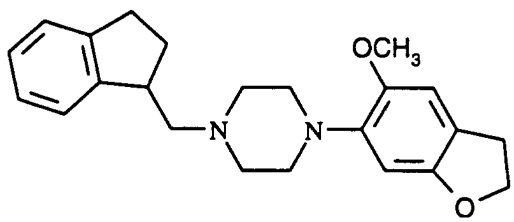

Préparée comme décrit dans l'exemple 9, à partir de l'acide indan-1-yl carboxylique et de la 4-(2,3-dihydro-5-méthoxy benzofuran-6-yl) pipérazine. Le fumarate du produit titre fond à 195-197°C (éthanol).

**Exemple 30 :**

4-(2,3-dihydrobenzofuran-6-yl)-1-(indan-2-yl méthyl) pipérazine

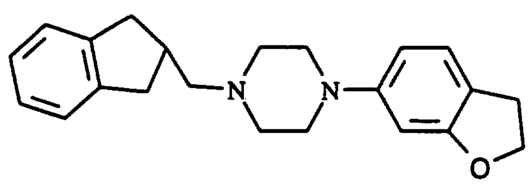

Préparée comme décrit dans l'exemple 9, à partir de la 4-(2,3-dihydrobenzofuran-6-yl) pipérazine. L'hémifumarate du produit titre fond à 171-173°C (éthanol).

**Exemple 31:**

4-(2,3-dihydro benzo-1,4-dioxin-6-yl)-1-[(indan-2-yl)méthyl]-1,2,3,6-tétrahydropyridine

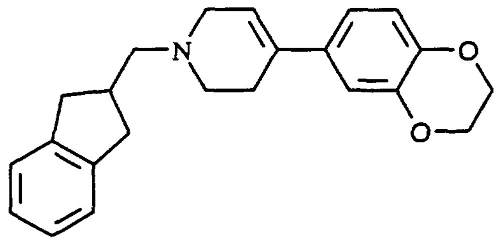

*Stade 1 : "Amide"*
Identique au stade 1 de l'exemple 6, mais en utilisant la 4-(2,3-dihydro-benzo-1,4-dioxin-6-yl)-1,2,3,6-tétrahydropyridine (Préparation 3) à la place de la 4-(2,3-dihydro-benzo-1,4-dioxin-6-yl) pipérazine.

*Stade 2 : Produit titre*
A une solution de 1,9 g (5,2 mmole) de l'amide ci-dessus préparée, dans 60 ml de toluène, on coule goutte à goutte 4,4 ml (15,8 mmole) de Red-Al® 3,5 M dans le toluène. On chauffe 2 heures à 50°C puis on laisse agiter la nuit à température ambiante. On refroidit ensuite l'ensemble au bain de glace et on hydrolyse successivement par 2,2 ml d'éthanol puis 2,6 ml d'eau. On filtre les sels d'aluminium et évapore à sec le filtrat. On obtient 1,4 g d'une huile que l'on purifie par flash-chromatographie.
Le fumarate du produit titre fond à 160-167°C (éthanol).
Rendement : 25%

**Exemple 32 :**

4-(2,3-dihydro-benzo-1,4-dioxin-6-yl)-1-[2-(napht-1-yl)éthyl]-1,2,3,6-tétrahydropyridine

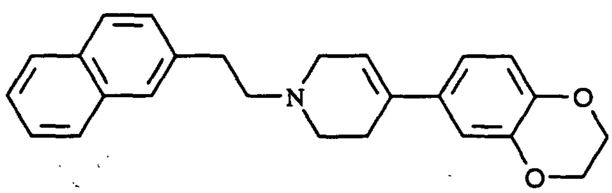

Préparée comme décrit dans l'exemple 28 mais en utilisant au stade 1 l'acide napht-1-yl acétique à la place de l'acide indan-1-yl carboxylique. Le fumarate du produit titre fond à 170-180°C (éthanol).

**Exemple 33 :**

4-(2,3-dihydro-5-méthoxy benzofuran-6-yl)-1-[2-1,2-dihydronaphthalén-3-yl)méthyl]pipérazine

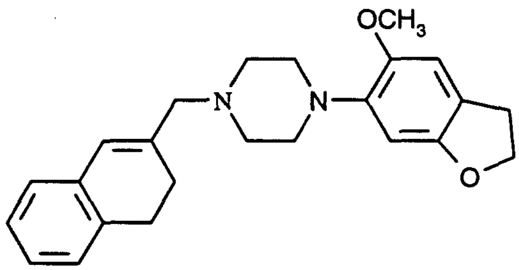

Préparée comme décrit dans l'exemple 31 mais en utilisant au stade 1 l'acide 1,2-dihydronaphtalén-3-yl carboxylique et la 4-(2,3-dihydro-5-méthoxy benzofuran-6-yl) pipérazine. Le fumarate du produit titre fond à 180-184°C (éthanol).

**Exemple 34 :**

1-(indan-2-yl méthyl)-4-(5-méthoxy benzofuran-6-yl) pipérazine

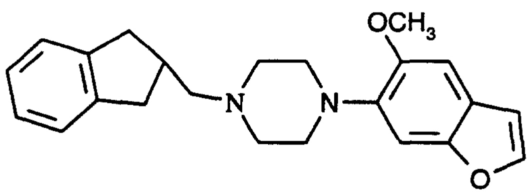

Préparée comme décrit dans l'exemple 31 mais en utilisant au stade 1 la 4-(5-méthoxy benzofuran-6-yl) pipérazine. Le fumarate du produit titre fond à 188-192°C (éthanol).

**Exemple 35 :**

4-(benzofuran-6-yl)-1-(indan-2-yl méthyl) pipérazine

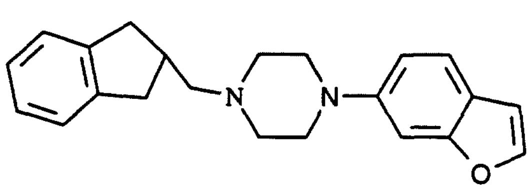

Préparée comme décrit dans l'exemple 31 mais en utilisant au stade 1 la 4-(benzofuran-6-yl) pipérazine. Le difumarate du produit titre fond à 168-170°C (éthanol).

**Préparation des matières premières nouvelles**

**Préparation 1.**

**4-(2,3-dihydro-5-méthoxy benzofuran-6-yl) pipérazine**

*Stade 1. 2,3-dihydro-5-méthoxy-6-nitrobenzofurane.*

A 18,7 ml d'acide nitrique fumant dans 37,5 ml d'eau, on ajoute goutte à goutte à 0° C, en 15 mn, 15 g (100 mM) de 2,3-dihydro-5-méthoxybenzofurane en solution dans 15 ml . d'acide acétique glacial. On agite 1h à 0° C puis lh30 à température ambiante. On verse le milieu réactionnel dans 125 ml d'eau, filtre le solide obtenu et le rince abondamment à l'eau. Après séchage on recueille 16,9 g du produit attendu. (Rdt = 87 %). P.F. = 108 - 109° C.

*Stade 2. 6-amino-2,3-dihydro-5-méthoxy-benzofurane.*

7,1 g (36,4 mM) du composé obtenu au stade précédent dans 100 ml de méthanol contenant 100 mg d'oxyde de platine sont hydrogénés à température ambiante et pression ordinaire pendant 3h. Après filtration du catalyseur et évaporation du solvant on recueille 5,85 g de l'amine attendue sous forme d'huile (Rdt = 97 %).

*Stade 3. Produit titre.*

5,75 g (34,8 mM) de l'amine obtenue ci-dessus, 6,2 g (34,8 mM) de chlorhydrate de bis (2-chloroéthyl) amine, et 4,81 g (34,8 mM) de carbonate de potassium en solution dans 90 ml de chlorobenzène sont portés à reflux pendant 22h. On verse dans l'eau et décante le chlorobenzène. La phase aqueuse est basifiée par 12 ml de soude concentrée et extraite par 2 fois 250 ml d'acétate d'éthyle. Les phases organiques sont lavées par 250 ml d'une solution saturée en NaCl. Après séchage on obtient 5,95 g de produit attendu, purifié sous forme de son chlorhydrate. P.F. > 260° C.

**Préparation 2.**

**4-(2,3-dihydrobenzo-1,4-dioxin-6-yl)pipéridine**

*Stade 1. Magnésien du 6-bromo-2,3-dihydrobenzo-1,4-dioxine.*

On ajoute rapidement 10 g (46 mM) de 6-bromo-2,3-dihydrobenzo-1,4-dioxine en solution dans 100 ml de THF, à une suspension de 1,1 g (0,046 atome-gramme) de magnésium dans 20 ml de THF. On amorce la réaction en chauffant en présence d'un cristal d'iode et de quelques gouttes d' iodure de méthyle. Après la fin de l'addition (environ 15 minutes), on chauffe 1h à reflux. On obtient une solution parfaitement limpide.

*Stade 2. 1-benzyl 4-(2,3-dihydrobenzo-1,4-dioxin-6-yl) 4-hydroxy pipéridine.*

A la solution du réactif de Grignard obtenue ci-dessus on ajoute à 0° C, 6,9 g (37 mM) de N-benzyl pipérid-4-one en solution dans 80 ml de THF. Après la fin de l'addition on agite 2h à température ambiante, puis on hydrolyse avec une solution saturée de $NH_4Cl$. On concentre à sec puis reprend le résidu à l'éther et l'extrait avec de l'HCl 1N. La phase acide est basifiée à la soude N puis on extrait à l'éther. Cette phase éthérée est séchée puis évaporée à sec. On obtient 8 g de produit attendu (Rdt = 67 %). P.F. = 154 - 156° C.

*Stade 3. 1-benzyl-4-(2,3-dihydrobenzo-1,4-dioxin-6-yl)-1, 2, 3, 6-tétrahydropyridine.*

On ajoute 4 g (12,3 mM) de produit obtenu au stade 2 à 50 ml d'acide trifluoroacétique. On chauffe 30 min. à 60° C, refroidit et neutralise avec de la soude à 35 %. On extrait à l'éther, lave à l'eau, séche sur $MgSO_4$, filtre et évapore à sec. Le résidu obtenu est purifié par chromatographie sur silice (éluant : $CH_2Cl_2/CH_3OH$ : 95/5). On obtient 1,5 g du produit désiré sous forme d'huile (Rdt = 40 %).

*Stade 4. Produit titre.*

On dissout 3,2 g (10,4 mM) de produit obtenu au stade 3 dans 150 ml d'éthanol. On ajoute 1,5 g de Pd $(OH)_2$ à 20 % sur charbon et hydrogène à température ambiante pendant 24 h sous une pression de $4.10^5$ Pa. On filtre le catalyseur et évapore à sec. On obtient 1,7 g de produit attendu sous forme d'huile (Rdt = 77 %).

**Préparation 3.**

**4-(2,3-dihydrobenzo-1,4-dioxin-6-yl)1,2,3,6-tétrahydropyridine**

2 g (6,5 mmole) du composé obtenu au stade 3 de la préparation 2 sont dissous dans 30 ml de 1,2-dichloroéthane. On ajoute 1,2 ml (13 mmole) de chloroformiate d'éthyle puis on porte 2 heures à reflux. On refroidit et évapore

à sec. Le résidu est repris par 30 ml d'éthanol et 0,7 g (13 mmole) de potasse puis porté à reflux une nuit. On refroidit, rajoute 20 ml d'eau et encore 1,05 g (19,5 mmole) de potasse et porte de nouveau à reflux pendant deux jours. Après évaporation de l'éthanol, on dilue à l'eau et extrait à l'acétate d'éthyle. On sèche sur $MgSO_4$, filtre et évapore à sec pour obtenir 1,4 g du produit titre (Rdt : 100%)

**Préparation 4.**

**4-(2,3-dihydro-5-éthoxybenzofuran-6-yl) pipérazine**

Obtenue de la même façon que le produit de la préparation 1 mais en remplaçant au stade 1 le 2,3-dihydro-5-méthoxy-benzofurane par le 2,3-dihydro-5-éthoxy-benzofurane. Solide marron (P.F. = 68-69°C)

**Préparation 5.**

**4-(2,3-dihydro-7-méthoxybenzo-1,4-dioxin-6-yl)pipérazine**

Obtenue de la même façon que le produit de la préparation 1 mais en remplaçant au stade 1 le 2,3-dihydro-5-méthoxy-benzofurane par le 2,3-dihydro-7-méthoxy-benzo-1,4-dioxine. Le chlorhydrate de produit titre fond à 180-182°C.

**Préparation 6.**

**4-(8-méthoxy-benzo-1,5-dioxépin-7-yl)pipérazine**

Obtenue de la même façon que le produit de la préparation 1 mais en remplaçant au stade 1 le 2,3-dihydro-5-méthoxy-benzofurane par la 8-méthoxy-benzo-1,5-dioxépine. Le chlorhydrate du produit titre fond à 187-189°C.

**Préparation 7.**

**acide cyclopenta[c]thién-5-yl-carboxylique**

*Stade 1 : diester éthylique de l'acide cyclopenta[c]thiophene 5,5-dicarboxylique*

A température ambiante, on mélange 4,5 g (15,0 mmole) de 3,4-bis-(bromométhyl)thiophène (dont la synthèse est décrite dans J. Prakt. Chem. 1972, 314(2), 334-352), 2,3 ml (15,0 mmole) de malonate de diéthyle, 4,3 g (31,0 mmole) de carbonate de potassium et 75 ml de méthyléthylcétone. On porte à reflux pendant 20 heures, puis évapore à sec. On reprend par 200 ml de dichlorométhane et lave 2 fois par 50 ml d'eau. Après séchage sur sulfate de magnésium, puis évaporation, le résidu est chromatographié sur silice (éluant dichlorométhane) pour donner 1,8 g du composé désiré (Rdt : 45%).

*Stade 2 : diacide cyclopenta[c]thiophène 5,5-dicarboxylique*

A 2,6 g (9,7 mmole) du composé précédent dans 5 ml d'éthanol, on ajoute d'un trait une solution de 2,2 g (38,8 mmole) de potasse dans 2,2 ml d'eau. On porte à reflux 6h, puis évapore à sec. Le résidu est repris par 50 ml d'acide chlorhydrique N et extrait 3 fois par 80 ml d'éther. Les phases éthérées jointes sont séchées sur sulfate de magnésium, puis concentrées pour donner 1,85 g du composé désiré (Rdt 88%).

*Stade 3 : acide cyclopenta[c]thién-5-yl-carboxylique*

On porte à reflux pendant 1 heure, 1,8 g (8,5 mmole) du composé précédent dans 8,5 ml de N,N-diméthylacétamide. On évapore ensuite à sec, puis reprend par 100 ml d'éther et lave 4 fois par 50 ml d'eau. Après séchage sur sulfate de magnésium, puis évaporation, on recueille 1,32 g du composé désiré (Rdt : 94%).

**Préparation 8.**

**acide cyclopenta[b]thién-5-yl-carboxylique**

*Stade 1 : 3-(thién-3-yl) 3-oxo propanoate de méthyle*

A 25,2 g (0,2 mmole) de 3-acétyl thiophène dans 600 ml de carbonate de diméthyle, à 0°C, on ajoute par

fractions en 10mn 24 g (0,6 mmole) d'hydrure de sodium (à 60%), puis porte à reflux 30 mn On laisse refroidir et verse dans 1 litre d'un mélange eau/glace contenant 53 ml d'acide acétique. On extrait 3 fois par 250 ml d'éther. Les phases organiques jointes sont séchées sur sulfate de magnésium. Après évaporation , le résidu est chromatographié sur silice (éluant : dichlorométhane) pour donner 17 g du composé désiré (Rdt : 46%).

*Stade 2 : ester méthylique de l'acide 4-oxo cyclopenta[b]thién-5-yl-carboxylique*

A température ambiante, à 7,8 g (58,5 mmole) de chlorure d'aluminium dans 75 ml de nitrométhane, on ajoute 4,9 g (26,6 mmole) du céto-ester précédent, puis agite 15 mn. On ajoute alors 2,9 ml (31,9 mmole) de chlorure de 2-méthoxy acétyle dans 25 ml de nitrométhane, goutte à goutte en 10 mn, puis porte à 80°C pendant 3 heures. On laisse refroidir et verse dans 100 ml d'une solution aqueuse à 10 % d'acide oxalique, puis extrait 3 fois par 100 ml d'éther. Les phases éthérées jointes sont lavées 2 fois par 150 ml d'une solution aqueuse saturée d'hydrogénocarbonate de sodium, séchées sur sulfate de magnésium et concentrées, le résidu étant chromatographié sur silice pour donner 2,85 g du composé désiré (Rdt 55%).

*Stade 3 : ester méthylique de l'acide cyclopenta[b]thién-5-yl carboxylique*

Dans un mortier, on mélange intimement 7 g (107,2 atome-gramme) de zinc avec 0,78 g (2,9 mmol) de chlorure mercurique, puis agite énergiquement le tout dans 10 ml d'eau contenant 0,3 ml d'acide chlorhydrique concentré, pendant 10 mn. On décante la phase aqueuse, puis ajoute successivement 6 ml d'eau, 12 ml d'acide chlorhydrique concentré, puis 2,8 g (14,3 mmole) du composé précédent dans 15 ml de toluène. On porte à reflux pendant 18 heures, puis laisse refroidir, décante l'amalgame et extrait 2 fois par 20 ml d'éther. Les phases organiques jointes sont lavées 2 fois par 20 ml d'une solution aqueuse à 10% de carbonate de sodium, séchées sur sulfate de magnésium et concentrées, le résidu étant chromatographié sur silice (éluant : dichlorométhane + 2% d'acétate d'éthyle) pour donner 1,15 g du composé désiré (Rdt 44%).

*Stade 4 : acide cyclopenta[b]thién-5-yl carboxylique*

On agite à température ambiante pendant 24h, 1,05 g (5,8 mmole) du composé précédent et 3,5 ml (7 mmol) de soude 2N dans 6ml de méthanol, puis évapore à sec, reprend par 50 ml d'eau, lave 2 fois par 25 ml d'éther, acidifie par de l'acide chlorhydrique N et extrait 3 fois par 40 ml d'éther. Les phases éthérées jointes sont séchées sur sulfate de magnésium et concentrées pour donner 0,86 g du composé désiré (Rdt 88%)

**Exemple 30**

Etude pharmacologique

*In vitro Détermination de l'affinité pour les récepteurs humains $D_4$*

Les membranes préparées à partir de cellules CHO transfectées avec le récepteur humain $D_4$ ont été achetées auprès de Receptor Biology Inc. (MD, U.S.A.). Les membranes sont incubées en triple avec 30 µg de protéine membranaire, 0,5 mM de [$^3$H] spipérone et le ligand froid dans un volume final de 1 ml, pendant 60 minutes à 25° C. Le tampon d'incubation contient 50 mM de TRIS-HCl (pH 7.4), 120 mM de NaCl, 5 mM de KCl, 5mM de $MgCl_2$ et 1 mM d'EDTA. A la fin de l'incubation, le milieu d'incubation est filtré au travers de filtres WHATMAN® GF/B imprégnés avec 0,1 % de polyéthylénimine et lavés trois fois avec 2 ml de tampon refroidit. La radioactivité retenue sur les filtres est déterminée par comptage du liquide de scintilliation. Les isothermes de binding sont analysés par une méthode informatisée de régression non linéaire pour la détermination des valeurs d'$IC_{50}$. Elles sont converties en constante d'inhibition ($K_i$) par l'intermédiaire de l'équation de Cheng-Prusoff :

$$K_i = \frac{IC_{50}}{1 + L/Kd}$$

dans laquelle L est la concentration de [$^3$H] spipérone libre et Kd est la constante de dissociation de [$^3$H] spipérone du récepteur humain $D_4$ (70 pM).

Les produits de l'invention ont des valeurs de $K_i$ pour le récepteur $D_4$ inférieures à $5.10^{-8}$ M.

*Détermination de l'affinité pour les récepteurs humains $D_2$*

La procédure ici utilisée est déjà décrite en détail dans la littérature. Des cellules CHO sont transfectées de façon stable avec le cDNA codant pour le récepteur $D_2$ humain et pour les études de binding les membranes sont incubées avec 0,1 nM de [$^{125}$I]-iodosulpiride et le binding spécifique est supérieur à 90 %. Les $IC_{50}$ et $K_i$ sont déterminés et calculés comme ci-dessus. Les produits de l'invention ont des valeurs de $K_i$ pour le récepteur $D_2$ supérieures à $10^{-6}$ M.

*In vivo*

***a) modèles pharmacologiques***

Des rats Wistar males (Iffa Credo, Illskirchen France) de 250 à 280 g sont maintenus dans un cycle lumière/obscurité 12h/12h (lumière allumée à 7h30). Ils peuvent accéder librement à l'eau et à la nourriture ; la température de laboratoire est de 21 ± 1° C et l'humidité de 60 ± 5 %.

Turnover de dopamine : L'effet des produits de l'invention et du produit de référence sur le turnover de dopamine est déterminé après injection sous-cutanée. Après un intervalle de 30 minutes, les rats sont décapités et le cerveau est disséqué de façon à extraire le striatum, le noyau accumbens, le cortex frontal et les tubercules olfactifs. Les tissus sont homogénéisés dans 500 µl d'$HClO_4$ 0,1 M contenant 0,5 % de $Na_2S_2O_5$ et 0,5 % d'EDTA $Na_2$ et centrifugé à 15 000 g durant 15 minutes à 4° C. Les surnageants sont dilués dans la phase mobile et injectés dans une colonne HPLC (hypersil® ODS 5 µm, C18, 150 x 4,6 mm, Thermo Separation Products, Les Ulis, France) thermostatée à 25° C. La phase mobile HPLC est composée de 100 mM de $KH_2PO_4$ de 0,1 mM d'EDTA, de 0,5 mM d'octylsulphonate de sodium et de 5 % de méthanol ajustée à pH 3.15 avec $H_3PO_4$.
La phase mobile est injectée avec une pompe BECKMAN® 116 et à un débit de 1 ml/min. La détection électrochimique est effectuée par l'intermédiaire d'un détecteur Waters® M460 dont le potentiel de l'électrode de travail est de 850 mV par rapport à une référence Ag/AgCl. Les quantités de dopamine et d'acide dihydroxyphenyl acétique (DOPAC), métabolite de la dopamine, sont exprimées par rapport à la quantité de protéines contenues dans la structure cérébrale prélevée. Le sérum d'albumine de bovin (Sigma Chemical Co, St-Louis, MO) est utilisé comme référence. Le rapport DOPAC/dopamine est calculé et utilisé comme index de turnover.
Pour chaque expérience, le rapport entre la quantité moyenne (± S.E.M) de dopamine et de DOPAC est déterminé par rapport aux valeurs obtenues chez les animaux traités avec le véhicule (100 %).
L'activité des produits de l'invention et du produit de référence est exprimée par rapport à cette valeur témoin et rapportée à titre d'exemple dans le tableau ci-après.

| Rapport DA : DOPAC (% ± S.E.M) | | | | | |
|---|---|---|---|---|---|
| **Produits** | **Dose (mg/kg)** | **Cortex frontal** | **Noyau accumbens** | **Tubercules olfactifs** | **Striatum** |
| Véhicule | - | 100,0 ± 18,2 | 100,0 ± 7,8 | 100,0 ± 7,7 | 100,0 ± 2,2 |
| Halopéridol | 0,63 s.c | 232,1* ± 8,3 | 358,5* ± 15,6 | 298,5* ± 8,9 | 371,5* ± 17,8 |
| Exemple 6 | 40,0 s.c<br>160,0 p.o | 153,0* ± 10,4<br>167,2* ± 16,1 | 147,1* ± 9,5<br>195,4* ± 18,3 | 125,2 *± 1,6<br>155,2* ± 8,8 | 139,3* ± 8,4<br>197,1* ± 15,3 |
| N≥5 par valeur. | | | | | |

* p≤0,05 vs vehicule

Ces résultats montrent que, à l'instar de l'halopéridol, les composés de l'invention exercent un effet important sur la transmission dopaminergique au niveau de chacun des territoires étudiés indiquant une bonne activité in vivo et un bonne biodisponibilité par voie orale.

Dialyse : Les rats sont anesthésiés au pentobarbital (60 mg/kg i.p.). Ils sont placés dans un appareil stéréotaxique de Kopf et les guides de canules (guides intracérébraux, Carnegie Medicine, Stockholm, Suède) sont implantés soit dans le striatum et le noyau accumbens contralatéral, soit dans le cortex frontal cingulé suivant les coordonnées respectives décrites comme suit dans l'atlas de Paxinos et Watson (1982) : noyau accumbens (CMA/12, AP : + 1.6, L : ± 1.4, DV : - 5.7) ; striatum (CMA/12, AP : + 0.5, L : ± 2.8, DV : - 3) et cortex frontal cingulé (CMA/11, AP : + 2.2, L : ± 0.6, DV :- 0.2). Les rats sont mis en cage séparément et ne sont utilisés en dialyse que 5 jours plus tard. Le jour de la dialyse les sondes CMA/12 en polycarbonate (striatum : 3 mm de long, 0,5 mm de diamètre externe, noyau accumbens : 2 mm de long, 0,5 mm de diamètre externe) et des sondes CMA/11 en cuprophan® (cortex frontal cingulé : 4 mm de long, 0,24 mm de diamètre externe) sont descendues lentement et maintenues dans leur position. Ces sondes sont perfusées à un débit de 1 ml/min. avec une solution de 147,2 mM de NaCl, 4 mM de KCl et 2,3 mM de $CaCl_2$ amené à pH 7,3 avec un tampon phosphate (0,1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane froid. Des sections d'une épaisseur de 100 µm sont coupées et colorées avec du cresyl violet, ce qui permet la vérification de l'emplacement des sondes.
La quantification simultanée de dopamine, norépinéphrine et sérotonine est effectuée de la façon suivante : 20 µl

d'échantillons de dialyse sont dilués avec 20 µl de phase mobile ($NaH_2PO_4$ : 75 mM, EDTA : 20 µM, sodium dodecanesulphonate : 1 mM, méthanol : 17,5 %, triethylamine : 0,01 %, pH : 5,70) et 33 µl sont analysés par HPLC avec une colonne en phase inverse (hypersil® ODS 5 µm, C18, 150 x 4,6 mm, Thermo Séparation Products, les Ulis, France) thermostatée à 45° C et quantifiés par l'intermédiaire d'un détecteur coulométrique (ESA 5014, Coulochem II, Bedford, Mass., U.S.A.). Le potentiel de la première électrode du détecteur est fixée à - 90 mV (réduction) et la seconde à + 280 mV (oxydation). La phase mobile est injectée avec une pompe Beckman® 116 à un débit de 2 ml/min. Les limites de sensibilité pour la dopamine, la norépinéphrine et la sérotonine sont de 0,55 fmole par échantillon.
Tous les produits de l'invention et la substance de référence sont injectés par voie sous-cutanée dans un volume de 1.0 ml/kg. Les produits sont dissous dans de l'eau distillée additionnée de quelques gouttes d'acide lactique si nécessaire. Les quantités de neurotransmetteurs sont exprimées comme une fonction de la moyenne des 3 valeurs de base. Une analyse de variance, avec le facteur temps comme mesure repétée, suivi d'un test de Newman-Keuls (P < 0,05) est utilisée pour l'évaluation statistique des effets des produits.
L'activité des produits de l'invention et du produit de référence est exprimée par le pourcentage de variation de la quantité de neurotransmetteur après administration des produits en comparaison avec la valeur basale (= 100%).
A titre d'exemple nous rapportons dans le tableau ci-dessous les changements enregistrés au niveau de la quantité de dopamine.

| Moyenne ± S.E.M. en % | | | | |
|---|---|---|---|---|
| ***Produits*** | ***Dose (mg/kg)*** | ***Cortex frontal*** | ***Noyau accumbens*** | ***Striatum*** |
| Véhicule | - | 100,0 ± 14,3 | 100,0 ± 8,9 | 100,0 ± 5,6 |
| Haloperidol | 0,63 s.c. | 148* ± 9 | 133* ± 7 | 129* ± 7 |
| Exemple 6 | 40,0 s.c. | 201* ± 12 | 103* ± 6 | 104* ± 8 |
| N ≥ 5 par valeur | | | | |

* p < 0,05 vs vehicule

Ces résultats montrent que, contrairement au produit de référence, les produits de l'invention renforcent la transmission mésocorticale dopaminergique. Cet effet montre que les produits de l'invention permettent de contrôler plus efficacement les symptômes déficitaires de la schizophrénie et présentent également des propriétés antidepressives et pro-mnésiantes.

*b) modèles thérapeutiques*

**1. Verticalisation induite par l'Apomorphine (0.75 mg/kg, s.c.) chez la souris**

Ce test, décrit par Protais *et al* (Psychopharmacologie, 1976, 50, 1-6), permet d'évaluer l'activité antagoniste dopaminergique de produits antipsychotiques éventuels. Une souris ayant reçu de l'apomorphine et placée dans une cage à barreaux verticaux, reste la plupart du temps, immobile en haut de la cage, accrochée par les 4 pattes aux barreaux. Ce comportement de verticalisation est bloqué si un produit antagoniste dopaminergique a été administré avant l'apomorphine.
Test : dès l'administration sous-cutanée (s.c.) du produit ou du solvant (groupe contrôle) la souris est placée dans une cage grillagée cylindrique (14 cm diam. x 14 cm h), à barreaux verticaux. Trente minutes après, l'animal reçoit la dose d'apomorphine (0.75 mg/kg, s.c.). L'observation des animaux se fait à 10 et 20 minutes après l'injection d'apomorphine, avec attribution d'un *score 0* (4 pattes au sol), *score 1* (souris redressée, 2 pattes avant sur les barreaux) et *score 2* (souris accrochée par les 4 pattes sur les barreaux) à chaque temps de mesure. Le score de verticalisation utilisé pour les résultats est compris entre 0 et 4 (somme des 2 mesures). Chaque groupe expérimental comporte au moins 5 animaux.
Analyse statistique : L'effet du produit sur la verticalisation est évalué en comparant les scores obtenus dans chaque groupe ayant reçu une dose de produit, à ceux obtenus dans le groupe contrôle (solvant), par un test U de Mann et Whitney, avec un probabilité de P < 0.05. La Dose Inhibitrice 50 est la dose de produit qui diminue de moitié la moyenne des scores de verticalisation par rapport à celle du groupe contrôle.
Résultats : A titre d'exemple et pour illustrer l'effet des produits de l'invention, la dose inhibitrice 50, pour le composé de l'exemple 6 est de 3,88 mg/kg par voie sous-cutanée.

**2. Test d'agressivité chez des souris isolées.**

Ce test permet d'évaluer l'activité anti-agressive *intraspecies* d'un produit chez des souris qui ont été maintenues en isolement pendant plusieurs mois.

Animaux : Le test utilise des souris mâles CD (Charles River) de poids 22 à 25 g à leur arrivée dans l'animalerie. Dès leur arrivée, les animaux sont isolés dans des cages individuelles en polycarbonate opaque noir (23 x 14 x 13 cm) avec un couvercle grillagé et stabulés de façon chronique (6 mois environ) dans la pièce d'expérimentation.

Sélection des couples de souris : La sélection des couples de souris agressives qui seront utilisés de façon chronique dans l'étude, commence après un mois d'isolement des animaux. Une ou deux fois par semaine, on place dans la cage d'une souris (résidente), une souris d'une autre cage (intruse) et l'on observe si les deux animaux s'attaquent (reniflements, poursuites, mordillements, morsures) pendant cet essai. A la fin de l'essai (durée maximale de 10 min.), chaque souris est isolée à nouveau dans sa cage respective. Si il y a eu des attaques, le même couple sera testé à nouveau lors du prochain essai ; si il n'y a pas eu d'attaques, chaque souris de ce couple sera mise en présence d'une autre souris, lors de l'essai suivant. On sélectionne ainsi, au cours d'essais successifs, à raison de 1 ou 2 essais par semaine, les couples définitifs de souris qui seront utilisés pour les expériences. La sélection des couples est basée sur la stabilité de la combativité des animaux d'un essai à l'autre, la faible latence de la 1ère attaque et la fréquence et durée des attaques. Chez les couples ainsi sélectionnés, ces paramètres sont vérifiés chaque semaine au cours d'un essai rapide, sans traitement, deux jours avant le jour Test.

Test : Le test a lieu une fois par semaine. Trente minutes avant leur mise en présence, les deux souris du couple reçoivent chacune le même traitement (produit ou solvant) et restent isolés dans leur cage respective. A T0 min., la souris intruse est introduite dans la cage de la souris résidente pour une durée de 3 minutes. On note la latence (en sec) de la première attaque, le nombre et la durée totale (en sec) des attaques. On note aussi une inversion éventuelle de la dominance d'une souris par rapport à l'autre (en général, la souris résidente est la souris dominante). A la fin du test, la souris intruse retourne dans sa cage ; les animaux restent en isolement jusqu'au prochain essai rapide et test, la semaine suivante.

Analyse statistique :Les effets d'un produit sur l'agressivité sont évalués en comparant le nombre et la durée des attaques des couples ayant reçu le produit (groupes traités) à ceux obtenus chez les couples ayant reçu le solvant (groupe contrôle), en utilisant une analyse de variance (ANOVA) suivie d'un test de Dunnett's, avec probabilité de $P < 0.05$.

La Dose Inhibitrice 50 du nombre ou de la durée des attaques est la dose de produit qui réduit de moitié la moyenne de chacune de ces valeurs, par rapport à celle obtenue respectivement dans le groupe contrôle.

Résultats : A titre d'exemple et pour illustrer l'activité des produits de l'invention, la dose inhibitrice 50, pour le composé de l'exemple 6 est de 0,99 mg/kg par voie sous-cutanée.

**3. Induction de Catalepsies chez le Rat**

L'administration prolongée de neuroleptiques ou antipsychotiques "typiques" (haloperidol, chlorpromazine) chez des patients schizophrènes entraîne souvent l'apparition de signes extrapyramidaux (EPS) indésirables de type Parkinson, en particulier un phénomène d'immobilité (Davis *et al.*, 1983). Par contre, les antipsychotiques "atypiques" (clozapine) provoquent peu de signes extrapyramidaux.

Chez l'animal, l'administration aiguë d'antipsychotiques "typiques" induit une catalepsie, c'est-à-dire le maintien de l'animal dans une posture, souvent anormale, qui lui a été imposée par l'expérimentateur (Waldmeier, 1979). L'évaluation des propriétés cataleptogènes d'un produit chez le rat permet donc de savoir si ce produit administré chez l'Homme, risque de provoquer ou non, un syndrome de type extrapyramidal.

Test : les animaux sont placés en cage individuelle et mis à jeun la veille du test, avec boisson à volonté. Le test de catalepsie consiste à placer chaque patte arrière de l'animal sur la patte avant du même côté et à mesurer le temps (secondes), pendant lequel l'animal garde cette position "pattes croisées" (maximum 30 sec). Chaque animal est soumis à 3 essais successifs (un toutes les deux min.), l'animal étant retiré de sa cage et placé sur le plan de travail. Ces essais ont lieu 1 heure après injection sous-cutanée ou administration orale du produit ou de son solvant. La valeur moyenne des 3 essais représente la durée de catalepsie (sec) pour chaque animal. Il y a 5 à 6 rats par groupe expérimental.

Analyse statistique : L'effet du produit sur la durée de catalepsie est évalué par une ANOVA, suivie d'un test de Dunnett's, avec probabilité $P < 0.05$.

La dose active 50 d'induction de catalepsies est celle qui provoque une catalepsie d'une durée de 50 % par rapport à la valeur maximale de 30 sec (corrigée de la valeur du groupe contrôle solvant).

Résultats : A titre d'exemple et pour illustrer l'absence d'effet cataleptogène des composés de l'invention, le composé de l'exemple 6 a une dose active 50 supérieure à 80 mg/kg par voie sous-cutanée. Par comparaison, l'halopéridol, antipsychotique de référence, a une dose active 50 de 0,146 mg/kg par la même voie. Ce résultat démontre bien

le grand intérêt d'un blocage sélectif des récepteurs D4 par rapport aux récepteurs D2 pour éviter les effets secondaires de types extrapyramidaux rencontrés avec les antipsychotiques dont le mécanisme d'action repose entre autre sur un blocage des récepteurs D2.

## Revendications

**1.** Les composés de formule I :

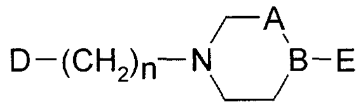

(I)

dans laquelle

A-B représente : $CH_2$-CH, CH=C ou $CH_2$-N,
n représente zéro ou un nombre entier de 1 à 6 inclus,
D représente l'un des systèmes bicycliques suivants :

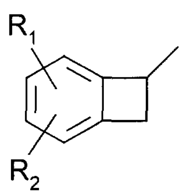 , 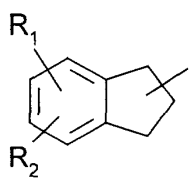 , 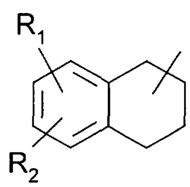 , 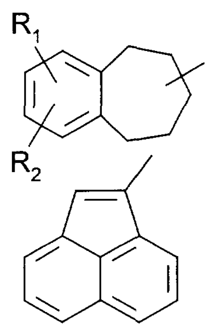 , 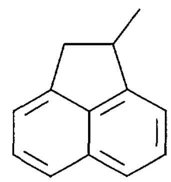

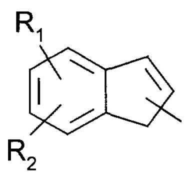 , 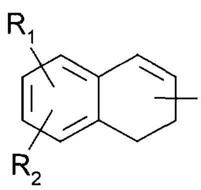 , 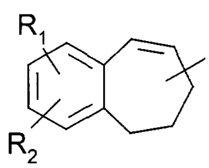 , , 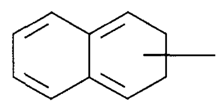 ,

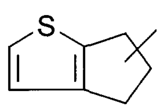 , 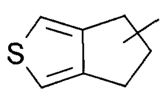 , 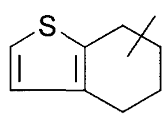 ,

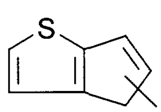 , 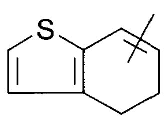 et 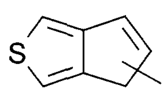

dans lesquels

$R_1$ et $R_2$, identiques ou différents, représentent chacun un atome d'hydrogène ou d'halogène, un radical alkyle ou alkoxy en chaîne droite ou ramifiée ayant de 1 à 5 atomes de carbone inclus ou un radical hydroxy ; et
E représente un des hétérocycles suivants :

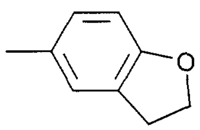 , 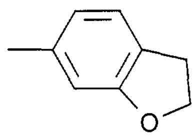 , 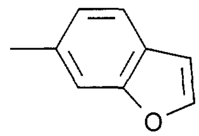 ,

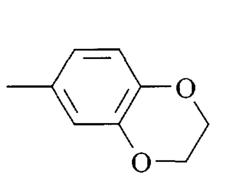 , 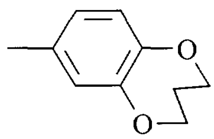 , 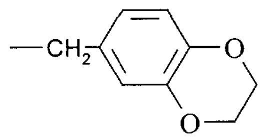 , 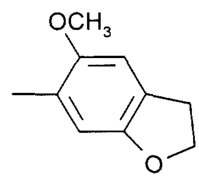 ,

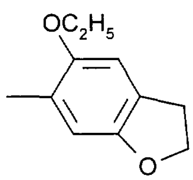 , 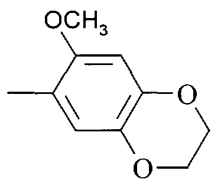 , 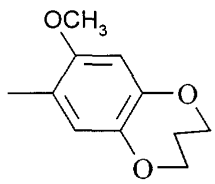 et 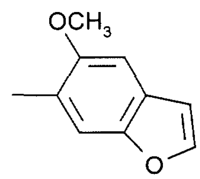 ,

à la condition toutefois que E ne représente pas :

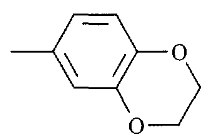

lorsque D représente :

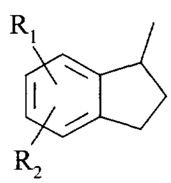 ,

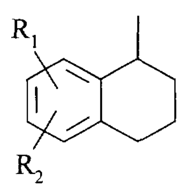 ou 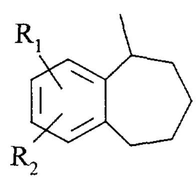 .

- leurs formes de mélange racémique ou racémate et d'isomères optiques ou énantiomères,
- ainsi que leurs sels avec des acides pharmaceutiquement acceptables.

**2.** Un composé de la revendication 1 qui est :
la 1-[2-(benzocyclobutan-1-yl)éthyl]-4-(2,3-dihydro-5-méthoxy benzofuran-6-yl)pipérazine, et son fumarate.

**3.** Un composé de la revendication 1 qui est :

la 4-(2,3-dihydrobenzo-1,4-dioxin-6-yl)-1-(indan-2-yl méthyl)pipérazine, et son dichlorhydrate.

**4.** Un composé de la revendication 1 qui est :
la 1-(indan-2-yl méthyl)-4-(2,3-dihydro-5-méthoxy benzofuran-6-yl)pipérazine, et son chlorhydrate.

**5.** Un composé de la revendication 1 qui est :
la 4-(2,3-dihydrobenzo-1,4-dioxin-6-yl)-1-(1,2,3,4-tétrahydronaphthalén-2-yl méthyl) pipérazine.

**6.** Un composé de la revendication 1 qui est :
la 4-(2,3-dihydrobenzofuran-5-yl)-1-(indan-2-yl méthyl) pipérazine.

**7.** Le procédé de préparation des composés de la revendicaiton 1 caractérisé en ce que l'on condense

- un composé de formule II :

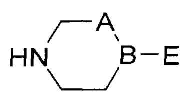

(II)

dans laquelle A-B et E ont les significations définies dans la revendication 1,

- avec un composé de formule III :

$$D\text{-}(CH_2)n\text{-}X \qquad (III)$$

dans laquelle n et D ont les significations définies dans la revendication 1 et X représente un atome d'halogène, ou un radical mésyloxy ou tosyloxy.

**8.** Le procédé de préparation des composés de la revendication 1 répondant plus précisémment à la formule I':

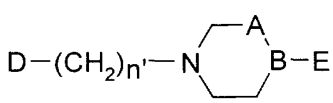

(I')

dans laquelle A-B, D et E ont les significations définies dans la revendication 1 et n' représente un nombre entier de 1 à 6, caractérisé en ce que l'on condense :

- un composé de formule II définie dans la revendication 7
  avec
- un composé de formule IV :

$$D\text{-}(CH_2)_{n'-1}\text{-}CO_2H \qquad (IV)$$

dans laquelle :

D et n' ont les significations définies ci-dessus,
et l'on réduit l'amide ainsi obtenue de formule V :

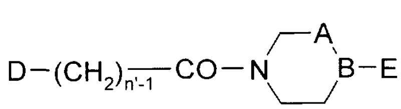

(V)

dans laquelle
A-B, D, E et n' ont les significations précédemment définies.

**9.** A titre de produits intermédiaires nouveaux formés dans la synthèse des composés de la revendication 1, les composés de formule V définie dans la revendication 8 dans lesquels D représente l'un des systèmes bicycliques suivants :

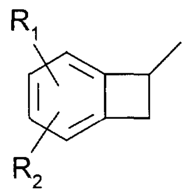 , 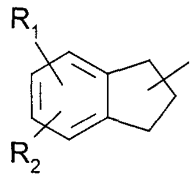 , 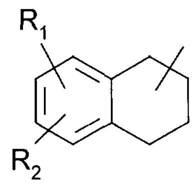 , 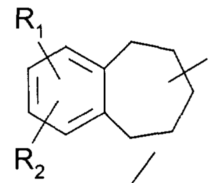 , 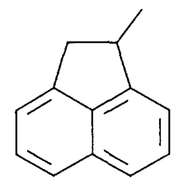

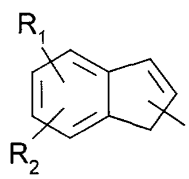 , 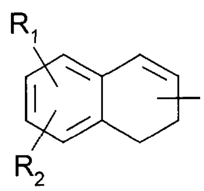 , 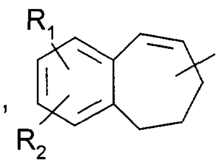 , 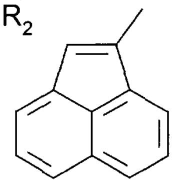 , 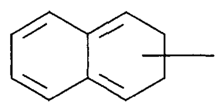 ,

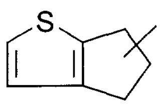 , 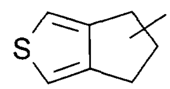 , 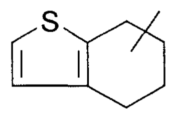 ,

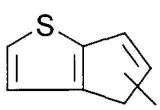 , 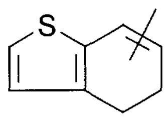 et 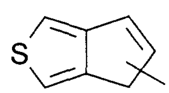

dans lesquels :

$R_1$ et $R_2$, identiques ou différents, représentent chacun un atome d'hydrogène ou d'halogène, un radical alkyle ou alkoxy en chaîne droite ou ramifiée ayant de 1 à 5 atomes de carbone inclus ou un radical hydroxy ; et E représente un des hétérocycles suivants :

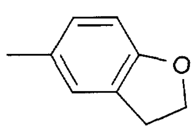 , 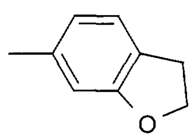 , 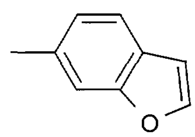 ,

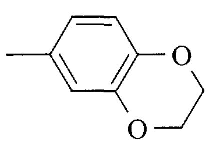 , 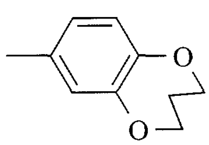 , 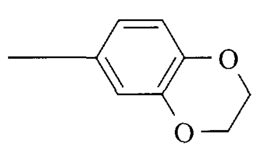 , 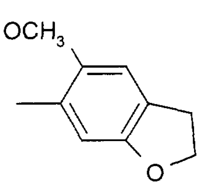 ,

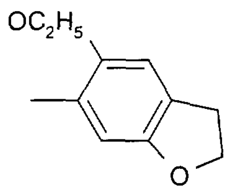 , 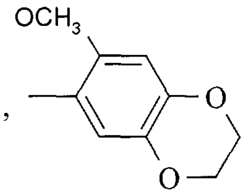 , 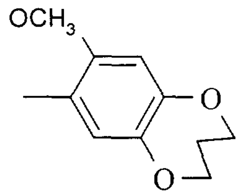 et 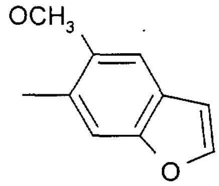 ,

à la condition toutefois que E ne représente pas :

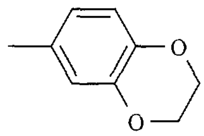

lorsque D représente :

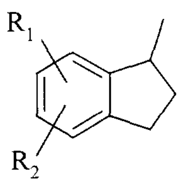 , 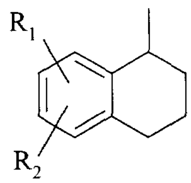 ou 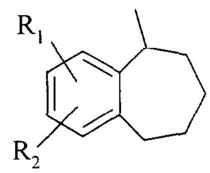 .

**10.** Les compositions pharmaceutiques, agissant comme ligands des récepteurs D4 contenant comme principe actif un composé selon l'une quelconque des revendications 1 à 6, avec un plusieurs excipients pharmaceutiquement appropriés.

**11.** Les compositions pharmaceutiques selon la revendication 10 présentées sous une forme convenant pour le traitement des maladies liées à un dysfonctionnement du système dopaminergique.

**Patentansprüche**

**1.** Verbindungen der Formel I:

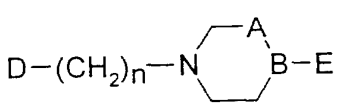 (I)

in der

A-B: $CH_2$-CH, CH=C oder $CH_2$-N,
n Null oder eine ganze Zahl mit einem Wert von 1 bis 6 einschließlich,
D eines der folgenden bicyclischen Systeme:

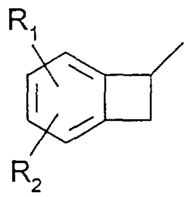 , 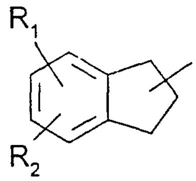 , 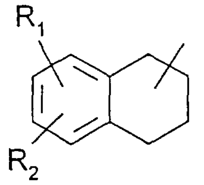 , 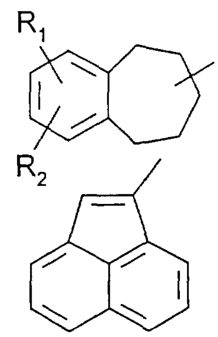 , 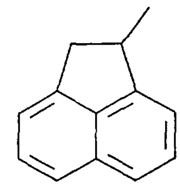

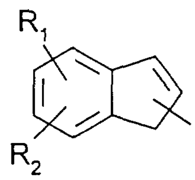 , 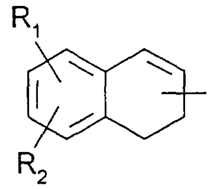 , 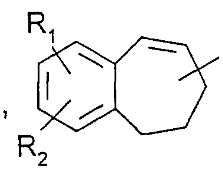 , , 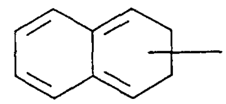 ,

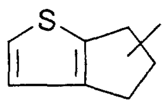 , 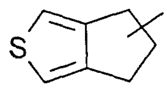 , 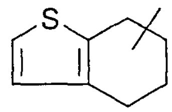 ,

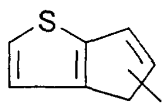 , 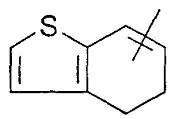 und 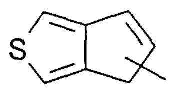

in denen:

$R_1$ und $R_2$, die gleichartig oder verschieden sind, jeweils ein Wasserstoffatom, ein Halogenatom, eine geradkettige oder verzweigte Alkyl- oder Alkoxygruppe mit 1 bis 5 Kohlenstoffatomen einschließlich oder eine Hydroxygruppe darstellen; und

E einen der folgenden Heterocyclen:

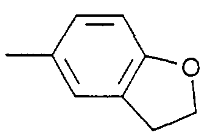 , 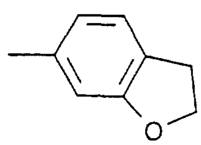 , 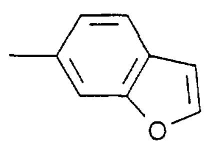 ,

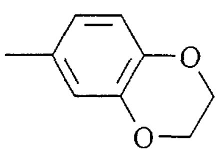 , 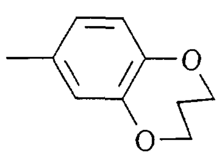 , 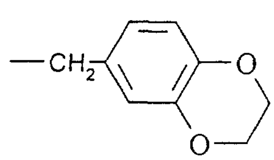 , 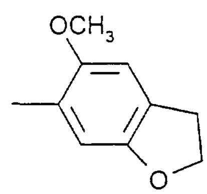 ,

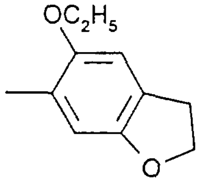 , 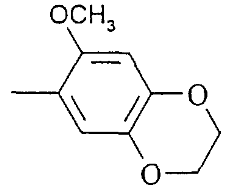 , 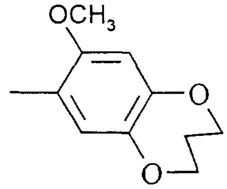 und 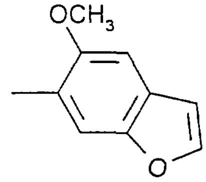 ,

mit der Maßgabe bedeuten, daß E nicht:

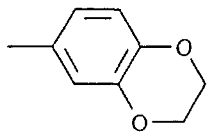

bedeutet, wenn
D die folgenden Bedeutungen besitzt:

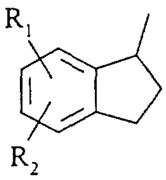,
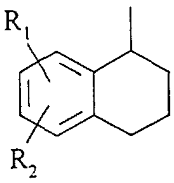
oder
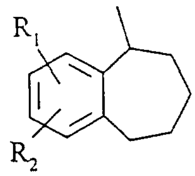

- deren racemische Mischungen, Racemate, optische Isomeren und Enantiomeren,
- sowie deren Salze mit pharmazeutisch annehmbaren Säuren.

**2.** Verbindung nach Anspruch 1, nämlich:
1-[2-(Benzocyclobutan-1-yl)-ethyl]-4-(2,3-dihydro-5-methoxy-benzofuran-6-yl)-piperazin und dessen Fumarat.

**3.** Verbindung nach Anspruch 1, nämlich:
4-(2,3-Dihydrobenzo-1,4-dioxin-6-yl)-1-(indan-2-yl-methyl)-piperazin und dessen Dihydrochlorid.

**4.** Verbindung nach Anspruch 1, nämlich:
1-(Indan-2-yl-methyl)-4-(2,3-dihydro-5-methoxy-benzofuran-6-yl)-piperazin und dessen Hydrochlorid.

**5.** Verbindung nach Anspruch 1, nämlich:
4-(2,3-Dihydrobenzo-1,4-dioxin-6-yl)-1-(1,2,3,4-tetrahydronaphthalin-2-yl-methyl)-piperazin.

**6.** Verbindung nach Anspruch 1, nämlich:
4-(2,3-Dihydrobenzofuran-5-yl)-1-(indan-2-yl-methyl)-piperazin.

**7.** Verfahren zur Herstellung der Verbindungen nach Anspruch 1, dadurch gekennzeichnet, daß man

- eine Verbindung der Formel II:

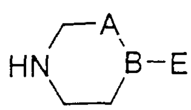
(II)

in der A-B und E die in Anspruch 1 angegebenen Bedeutungen besitzen,
- mit einer Verbindung der Formel III kondensiert:

$$D - (CH_2)_n - X \quad (III)$$

in der n und D die in Anspruch 1 angegebenen Bedeutungen besitzen und X ein Halogenatom oder eine Mesyloxy- oder Tosyloxygruppe bedeutet.

**8.** Verfahren zur Herstellung der Verbindungen nach Anspruch 1, namentlich der Formel I':

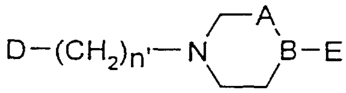 (I')

in der A-B, D und E die in Anspruch 1 angegebenen Bedeutungen besitzen und n' eine ganze Zahl mit einem Wert von 1 bis 6 bedeutet, dadurch gekennzeichnet, daß man:

- eine Verbindung der Formel II, wie sie in Anspruch 7 definiert ist, mit
- einer Verbindung der Formel IV:

$$D - (CH_2)_{n'-1} - CO_2H \qquad (IV)$$

in der:
D und n' die oben angegebenen Bedeutungen besitzen, kondensiert und das in dieser Weise erhaltene Amid der Formel V:

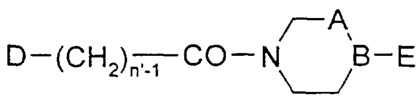 (V)

in der
A-B, D, E und n' die oben angegebenen Bedeutungen besitzen, reduziert.

**9.** Als neue Zwischenprodukte der Synthese der Verbindungen nach Anspruch 1, die Verbindungen der Formel V, wie sie in Anspruch 8 definiert sind, worin D eines der folgenden bicyclischen Systeme:

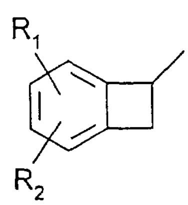 , 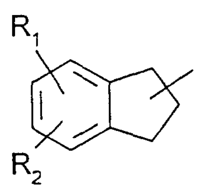 , 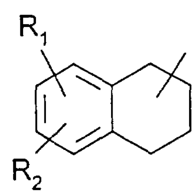 , 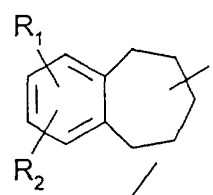 , 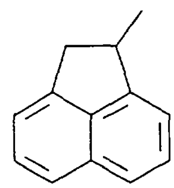

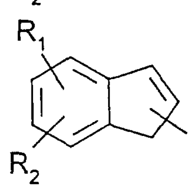 , 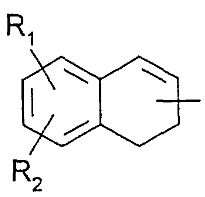 , 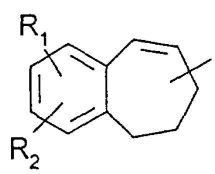 , 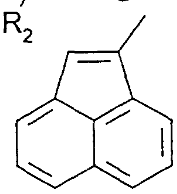 , 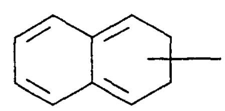 ,

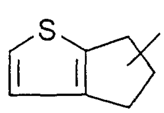 , 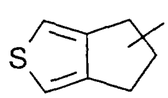 , 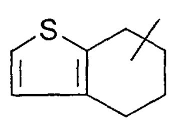 ,

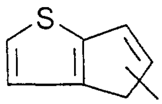 , 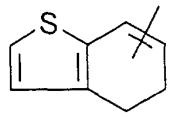 und 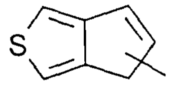

in denen:

$R_1$ und $R_2$, die gleichartig oder verschieden sind, jeweils ein Wasserstoff- oder ein Halogenatom, eine geradkettige oder verzweigte Alkyl- oder Alkoxygruppe mit 1 bis 5 Kohlenstoffatomen einschließlich oder eine Hydroxygruppe darstellen; und

E einen der folgenden Heterocyclen:

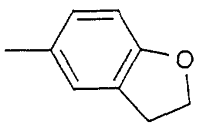 , 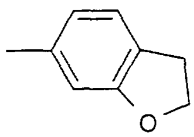 , 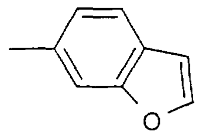 ,

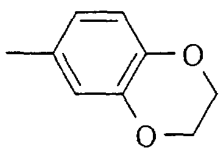 , 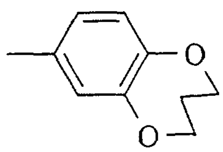 , 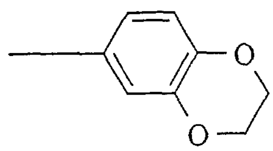 , 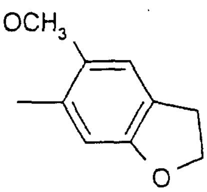 ,

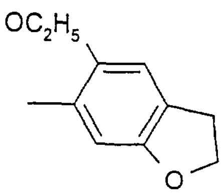 , 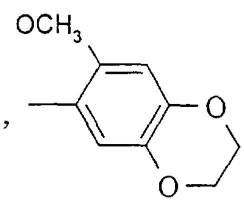 , 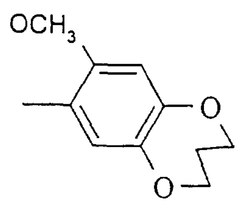 und 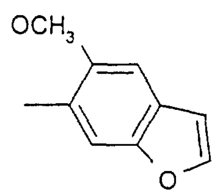 ,

mit der Maßgabe bedeuten, daß E nicht:

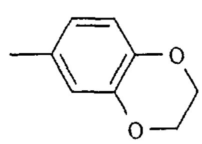

bedeutet, wenn D:

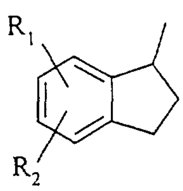 , 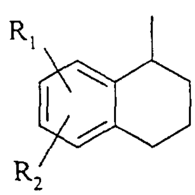 oder 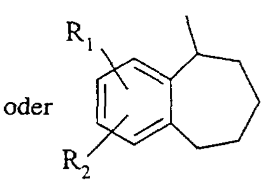

darstellt.

**10.** Pharmazeutische Zubereitungen, die als Liganden für die Rezeptoren D4 wirken, enthaltend als Wirkstoff eine Verbindung nach einem der Ansprüche 1 bis 6 zusammen mit einem oder mehreren pharmazeutisch geeigneten Trägermaterialien.

**11.** Pharmazeutische Zubereitungen nach Anspruch 10 in einer für die Behandlung von Erkrankungen, die mit einer Dysfunktion des dopaminergischen Systems verbunden sind, geeigneten Form.

**Claims**

**1.** Compounds of formula I:

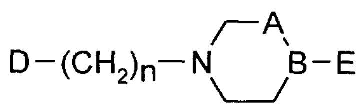

(I)

wherein

A-B represents : $CH_2$-CH, CH=C or $CH_2$-N,
n represents zero or an integer of from 1 to 6 inclusive,
D represents one of the following bicyclic systems :

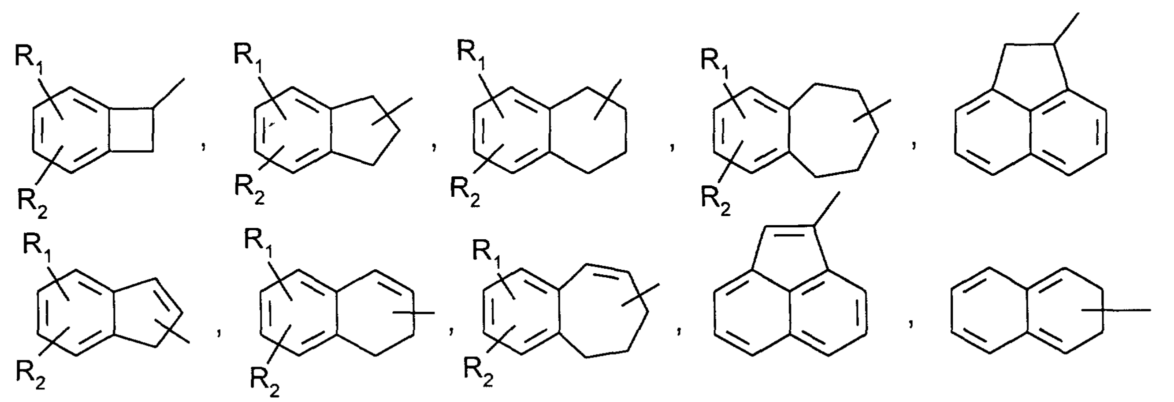

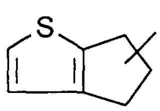 , 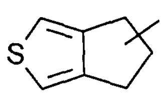 , 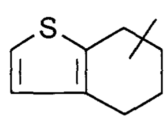 ,

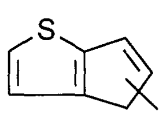 , 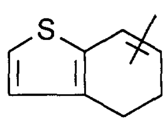 and 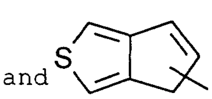

wherein :

$R_1$ and $R_2$, which are identical or different, each represents a hydrogen or halogen atom, a straight-chain or branched alkyl or alkoxy radical having from 1 to 5 carbon atoms inclusive or a hydroxy radical; and
E represents one of the following heterocycles :

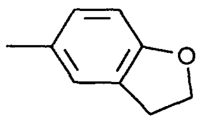 , 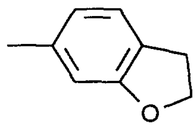 , 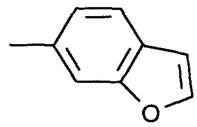 ,

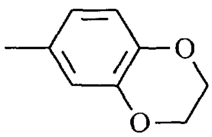 , 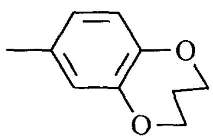 , 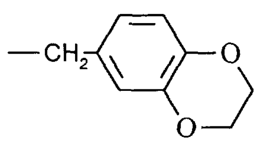 , 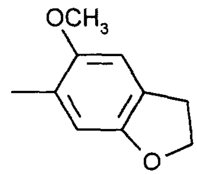 ,

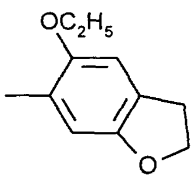 , 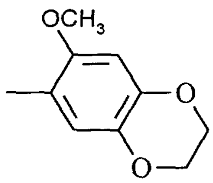 , 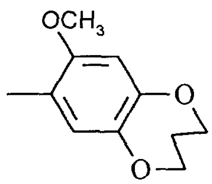 and 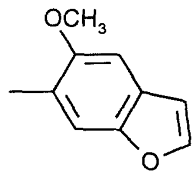 ,

but with the proviso that E does not represent:

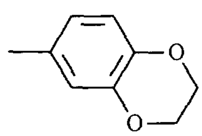

when D represents :

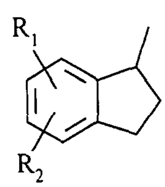 ,

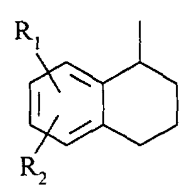 or 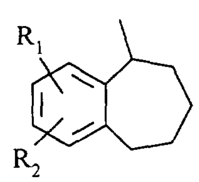 .

- their racemic mixture or racemate and optical isomer or enantiomer forms
- and also the salts thereof with pharmaceutically acceptable acids.

**2.** A compound according to claim 1 which is :
1-[2-(benzocyclobutan-1-yl)ethyl]-4-(2,3-dihydro-5-methoxybenzofuran-6-yl)piperazine, and its fumarate.

**3.** A compound according to claim 1 which is :
4-(2,3-dihydrobenzo-1,4-dioxin-6-yl)-1-(indan-2-ylmethyl)piperazine, and its dihydrochloride.

**4.** A compound according to claim 1 which is :
1-(indan-2-ylmethyl)-4-(2,3-dihydro-5-methoxybenzofuran-6-yl)piperazine, and its hydrochloride.

**5.** A compound according to claim 1 which is :
4-(2,3-dihydrobenzo-1,4-dioxin-6-yl)-1-(1,2,3,4-tetrahydronaphthalen-2-ylmethyl)-piperazine.

**6.** A compound according to claim 1 which is :
4-(2,3-dihydrobenzofuran-5-yl)-1-(indan-2-ylmethyl)piperazine.

**7.** A process for the preparation of compounds of claim 1, characterised in that

- a compound of formula II:

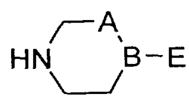

(II)

wherein A-B and E are as defined in claim 1, is condensed
- with a compound of formula III :

$$D\text{-}(CH_2)n\text{-}X \qquad (III)$$

wherein n and D are as defined in claim 1 and X represents a halogen atom or a mesyloxy or tosyloxy radical.

**8.** A process for the preparation of compounds of claim 1 corresponding more precisely to formula I':

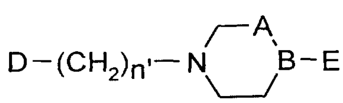

(I')

wherein A-B, D and E are as defined in claim 1 and n' represents an integer of from 1 to 6, characterised in that:

- a compound of formula II defined in claim 7 is condensed with
- a compound of formula IV :

$$D\text{-}(CH_2)_{n'-1}\text{-}CO_2H \qquad (IV)$$

wherein :
D and n' are as defined above,
and the amide so obtained of formula V :

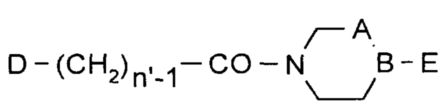

(V)

wherein
A-B, D, E and n' are as defined above, is reduced.

**9.** As new intermediate products formed in the synthesis of the compounds of claim 1, the compounds of formula V defined in claim 8 wherein D represents one of the following bicyclic systems:

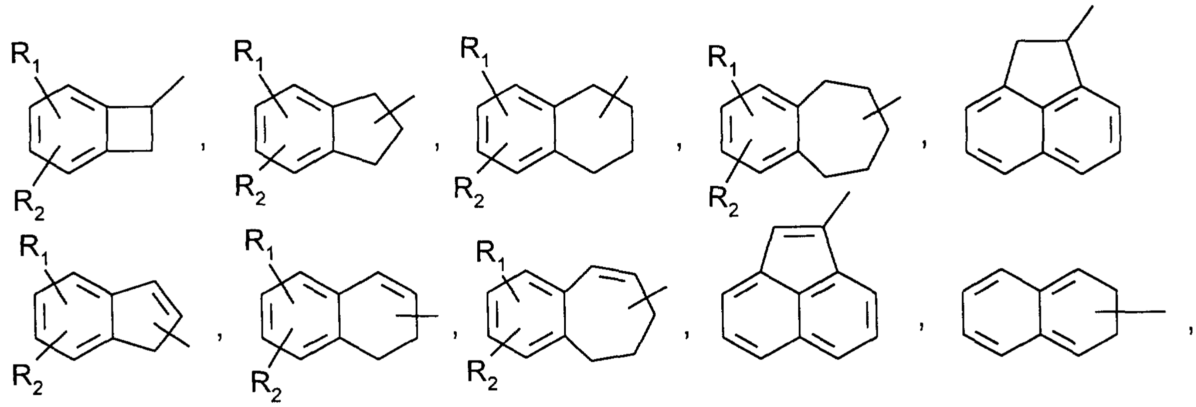

, , , , , , , , ,

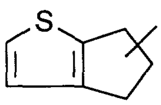 , 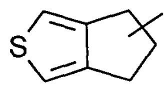 , 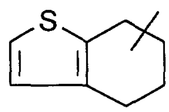 ,

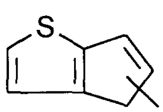 , 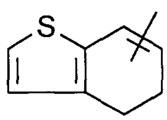 and 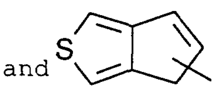

wherein :

$R_1$ and $R_2$, which are identical or different, each represents a hydrogen or halogen atom, a straight-chain or branched alkyl or alkoxy radical having from 1 to 5 carbon atoms inclusive or a hydroxy radical; and

E represents one of the following heterocycles :

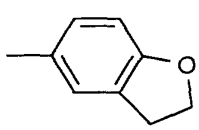 , 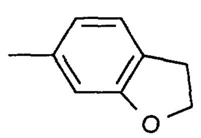 , 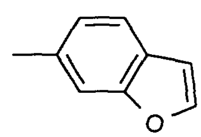 ,

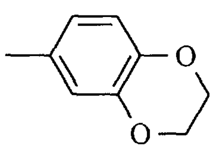 , 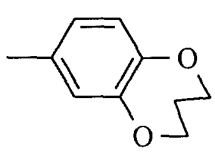 , 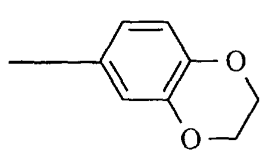 , 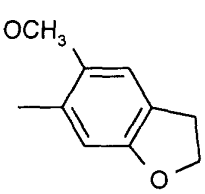 ,

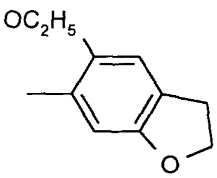 , 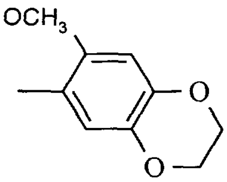 , 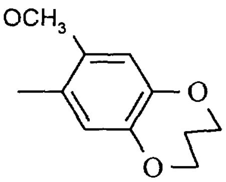 and 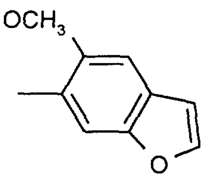 ,

but with the proviso that E does not represent:

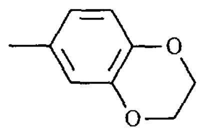

when D represents :

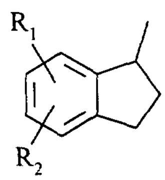 ,

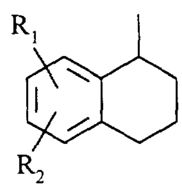 or 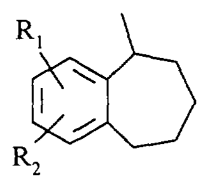 .

**10.** Pharmaceutical compositions acting as D4 receptor ligands, comprising as active ingredient a compound according to any one of claims 1 to 6, together with one or more pharmaceutically appropriate excipients.

**11.** Pharmaceutical compositions according to claim 10, presented in a form suitable for the treatment of disorders associated with a dysfunction of the dopaminergic system.